(19) Europäisches Patentamt / European Patent Office / Office européen des brevets

(11) **EP 3 598 327 B1**

## (12) EUROPEAN PATENT SPECIFICATION

(45) Date of publication and mention
of the grant of the patent:
**05.05.2021 Bulletin 2021/18**

(51) Int Cl.:
*G16B 20/00* (2019.01)  *G16B 30/10* (2019.01)

(21) Application number: **18305994.8**

(22) Date of filing: **20.07.2018**

(54) **METHOD AND ELECTRONIC SYSTEM FOR PREDICTING AT LEAST ONE FITNESS VALUE OF A PROTEIN VIA AN EXTENDED NUMERICAL SEQUENCE, RELATED COMPUTER PROGRAM PRODUCT**

VERFAHREN UND ELEKTRONISCHES SYSTEM ZUR VORHERSAGE VON MINDESTENS EINEM FITNESSWERT EINES PROTEINS MITTELS EINER ERWEITERTEN NUMERISCHEN SEQUENZ, ENTSPRECHENDES COMPUTERPROGRAMMPRODUKT

PROCÉDÉ ET SYSTÈME ÉLECTRONIQUE PERMETTANT DE PRÉDIRE AU MOINS UNE VALEUR D'APTITUDE D'UNE PROTÉINE À L'AIDE D'UNE SÉQUENCE NUMÉRIQUE AUGMENTÉE, PRODUIT LOGICIEL ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.01.2020 Bulletin 2020/04**

(73) Proprietor: **PEACCEL**
**97490 Sainte-Clotilde (FR)**

(72) Inventors:
• **CADET, Xavier**
**75013 PARIS (FR)**
• **FONTAINE, Nicolas**
**97419 la Possession (RE)**

(74) Representative: **Lavoix**
**2, place d'Estienne d'Orves**
**75441 Paris Cedex 09 (FR)**

(56) References cited:
**EP-A1- 3 082 056      WO-A1-2008/129458**
**US-A1- 2004 029 126**

• **COSIC I: "MACROMOLECULAR BIOACTIVITY: IS IT RESONANT INTERACTION BETWEEN MACROMOLECULES? - THEORY AND APPLICATIONS", IEEE TRANSACTIONS ON BIOMEDICAL ENGINEERING, IEEE SERVICE CENTER, PISCATAWAY, NJ, USA, vol. 41, no. 12, 1 December 1994 (1994-12-01), pages 1101-1114, XP000556764, ISSN: 0018-9294, DOI: 10.1109/10.335859**

## Description

The present invention concerns a method and a related electronic system for predicting at least one fitness value of a protein, the protein comprising an amino acid sequence. The invention also concerns a computer program product including software instructions which, when implemented by a computer, implement such a method.

## Background of the invention

Proteins are biological molecules consisting of at least one chain of amino acids sequence. Proteins differ from one another primarily in their sequence of amino acids, the differences between sequences being called "mutations".

One of the ultimate goals of protein engineering is the design and construction of peptides, enzymes, proteins or amino acid sequences with desired properties (collectively called "fitness"). The construction of modified amino acid sequences with engineered amino acid substitutions, deletions or insertions of amino acids or blocks of amino acids (chimeric proteins) (i.e. "mutants") allows an assessment of the role of any particular amino acid in the fitness and an understanding of the relationships between the protein structure and its fitness.

The main objective of the quantitative structure-function/fitness relationship analysis is to investigate and mathematically describe the effect of the changes in structure of a protein on its fitness. The impact of mutations is related to physico-chemical and other molecular properties of varying amino acids and can be approached by means of statistical analysis.

Exploring the fitness landscape, investigating all possible combinations (permutations) of n single point substitutions is a very difficult task. Indeed, the number of mutants increases very quickly (Table 1).

Table 1. Number of possible mutants for n mutations

| N° of single point mutations | N° of mutants |
| --- | --- |
| 2 | 4 |
| 4 | 16 |
| 6 | 64 |
| 8 | 256 |
| 10 | 1024 |
| 12 | 4096 |
| 14 | 16384 |
| 16 | 65536 |
|  |  |
| 40 | $1.1 \times 10^{12}$ |

Exploring all possible mutants is difficult experimentally, in particular when n increases. In practice, it is quite easy and cheap to produce mutants with single point substitutions in wet lab. For each of them, fitness can be readily characterized.

But combining single point substitutions is not so easy in wet lab. Generating all possible ($>=2^n$) combinations of targeted n single point substitutions can be very fastidious and costly. Evaluating fitness on large scale is problematic.

Mixed *in vitro and in silico* approaches have been developed to assist the process of directed evolution of proteins. They require from the wet lab to construct a library of mutants (by site-directed, random, or combinatorial mutagenesis), to retrieve the sequences and/or structures of a limited number of samples from library (called the "learning data set") and to assess fitness of each sampled mutant. They further require from the *in silico* to extract descriptors for each mutant, to use multivariate statistical method(s) for establishing relationship between descriptors and fitness (learning phase) and to establish a model to make predictions for mutants which are not experimentally tested.

A method based on 3D structure called Quantitative structure-function relationships (QFSR) has been proposed (Damborsky J, Prot. Eng. (1998) Jan;11(1):21-30). Other methods, based only on sequence, not on 3D structure, and performing *in silico* rational screening using statistical modelling were proposed (Fox R. et al., Protein Eng. (2003) 16(8):589-97; Fox R., Journal of Theoretical Biology (2005), 234:187-199; Minshull J. et al., Curr Opin Chem Biol. 2005 Apr;9(2):202-9; Fox R. et al., Nature Biotechnology (2007), 25(3):338-344; Fox R. and Huisman GW Trends Biotechnol. 2008 Mar;26(3):132-8). The most known is ProSAR (Fox R., Journal of Theoretical Biology (2005), 234:187-199; Fox R. et al., Nature Biotechnology (2007), 25(3):338-344) which is based on a binary encoding (0 or 1).

The QSFR method is efficient and takes into account information about possible interactions with non-variants residues. However, QSFR needs information on 3D protein structure, which is still currently limited, and the method is furthermore slow.

Comparatively, ProSAR does not need knowledge of 3D structure as it computed based on primary sequence only and can use linear and non-linear models. However, ProSAR still suffers from drawbacks and its capacity of screening is limited. In particular, only those residues undergoing variation are included in the modelling and, as a consequence, information about possible interactions between mutated residues and other non-variant residues are missing. ProSAR relies on binary encoding (0 or 1) of the mutations which does not take into account the physico-chemical or other molecular properties of the amino acids. Additionally, (i) the new sequences that can be tested are only sequences with mutations, or combinations of mutations, at the positions that were used in the learning set used to build the model; (ii) the number of positions of mutations in the new sequences to be screened cannot be different from the number of mutations in the train set; and (iii) the calculation time when introducing non-linear terms in order to build a model is very long on a super computer (up to 2 weeks for 100 non-linear terms).

A versatile and fast *in silico* approach to help in the process of directed evolution of proteins is therefore still needed. The invention provides a method fulfilling these requirements and which is based on Digital Signal Processing (DSP).

Digital Signal Processing techniques are analytic procedures, which decompose and process signals in order to reveal information embedded in them. The signals may be continuous (unending), or discrete such as the protein residues. In proteins, Fourier transform methods have been used for biosequence (DNA and protein) comparison, characterization of protein families and pattern recognition, classification and other structure based studies such as analysis of symmetry and repeating structural units or patterns, prediction of secondary/tertiary structure prediction, prediction of hydrophobic core, motifs, conserved domains, prediction of membrane proteins, prediction of conserved regions, prediction of protein subcellular location, for the study of secondary structure content in amino acids sequence and for the detection of periodicity in protein. More recently new methods for the detection of solenoids domains in protein structures were proposed.

Digital Signal Processing techniques have helped analyse protein interactions (Cosic I., IEEE Trans Biomed Eng. (1994) 41(12):1101-14) and made biological functionalities calculable. These studies have been reviewed in detail in Nwankwo N. and Seker H. (J Proteomics Bioinform (2011) 4(12): 260-268).

In these approaches, protein residues are first converted into numerical sequences using one of the available AAindex from the database AAindex (Kawashima, S. and Kanehisa, M. Nucleic Acids Res. (2000), 28(1):374; Kawashima, S. et al., Nucleic Acids Res. Jan 2008; 36), representing a biochemical property or physico-chemical parameter for each amino acid. These numerical sequences are then processed by means of Discrete Fourier Transform (DFT) to present the biological characteristics of the proteins in the form of Informational Spectrum. This procedure is called Informational Spectrum Method (ISM) (Veljkovic V, et al., IEEE Trans Biomed Eng. 1985 May;32(5):337-41). ISM procedure has been used to investigate principal arrangement in Calcium binding protein (Viari A, et al., Comput Appl Biosci. 1990 Apr;6(2):71-80) and Influenza viruses (Veljkovic V., et al. BMC Struct Biol. 2009 Apr 7;9:21, Veljkovic V., et al. BMC Struct Biol. 2009 Sep 28;9:62).

A variant of the ISM, which engages amino acids parameter called Electron-Ion Interaction Potential (EIIP) is referred as Resonant Recognition Model (RRM). In this procedure, biological functionalities are presented as spectral characteristics. This physico-mathematical process is based on the fact that biomolecules with same biological characteristics recognise and bio-attach to themselves when their valence electrons oscillate and then reverberate in an electromagnetic field (Cosic I., IEEE Trans Biomed Eng. (1994) 41(12):1101-14; Cosic I., The Resonant Recognition Model of Macromolecular Bioactivity Birkhauser Verlag,1997).

The Resonant Recognition Model involves four steps (see Nwankwo N. and Seker H., J Proteomics Bioinform (2011) 4(12): 260-268):

- Step 1: Conversion of the Protein Residues into Numerical Values of Electron-Ion Interaction Potential (EIIP) Parameter.
- Step 2: Zero-padding/Up-sampling. The process uses a zero padding to fill the gaps in the sequence of the proteins to be analysed at any position as signal processing requires that the window length of all proteins be the same.
- Step 3: processing of the Numerical Sequences using Fast Fourier Transform (FFT) to yield Spectral Characteristics (SC) and point-wise multiplied to generate the Cross Spectral (CS) features during step 4.
- Step 4: Cross-Spectral Analysis: Cross-Spectral (CS) analysis represents the point-wise multiplication of the Spectral Characteristics (SC).

Therefore, the CS analysis has been used qualitatively, to predict, for instance, ligand-receptor binding based on common frequencies (resonance) between the ligand and receptor spectra. Another example is to predict a ras-like activity or not, i.e. ability or not to transform cells, by applying the RRM to Ha-ras p21 protein sequence.

The information provided by these prior art methods are useful but are however insufficient to identify the most valuable protein mutants generated by directed evolution.

WO 2016/166253 A1 discloses a method and a related electronic system for predicting at least one fitness value of a protein, based on a protein spectrum, the protein spectrum being for example a Fourier Transform, such as a Fast Fourier Transform, applied to a numerical sequence obtained further to encoding the amino acid sequence of the protein.

The results provided by this last method are better than the ones provided by the other prior art methods.

However, the accuracy of the proteins fitness values predicted by this method may be further improved.

## Summary of the invention

The invention therefore relates to a method for predicting at least one fitness value of a protein, the method being implemented on a computer and including the following steps:

- calculating Q elementary numerical sequences, Q being an integer greater than or equal to 2, each elementary numerical sequence depending on a respective encoding of the amino acid sequence of the protein according to a protein database,
- determining an extended numerical sequence by concatenating the Q elementary numerical sequences,

for each fitness:

- comparing the determined extended numerical sequence with reference extended numerical sequences of a predetermined database, said database containing reference extended numerical sequences for different values of said fitness,
- predicting a value of said fitness according to the comparison step.

According to other advantageous aspects of the invention, the method comprises one or more of the following features taken alone or according to all technically possible combinations:

- at least one elementary numerical sequence is an elementary protein spectrum, the elementary protein spectrum being obtained by applying a Fourier Transform to an intermediate numerical sequence, the intermediate numerical sequence being obtained by a respective encoding of the amino acid sequence of the protein,

the Fourier Transform being preferably a Fast Fourier Transform,
at least one elementary protein spectrum being preferably calculated for said amino acid sequence according to a given set of frequency or frequencies;

- each elementary protein spectrum depends on the following equation:

$$f_j = \sum_{k=0}^{N-1} x_k \cdot exp\left(\frac{-2i\pi}{N} \cdot j \cdot k\right)$$

where j is an index-number of the elementary protein spectrum $f_j$;
the intermediate numerical sequence includes N value(s) denoted $x_k$, with $0 \leq k \leq N-1$ and $N \geq 1$; and
i defining the imaginary number such that $i^2 = -1$;

- the protein database includes at least one index of numerical values, each numerical value being given for a respective amino acid; and
wherein each encoding of the amino acid sequence of the protein is performed for a respective index, the value in the numerical sequence for each amino acid being equal to the numerical value for said amino acid in the respective index;
- all the elementary numerical sequences are distinct from each other;
- among a pair of elementary numerical sequences, one differs from the other further to the applying of the Fourier Transform for only one elementary numerical sequence of the pair and/or further to a different index from the one to the other elementary numerical sequence of the pair;

- the protein database includes several indexes of numerical values, and

wherein the method further includes a step of:

+ selecting the best index(es) based on a comparison of measured fitness values for sample proteins with predicted fitness values previously obtained for said sample proteins according to each index;
at least one encoding of the amino acid sequence of the protein being then performed using a respective selected index;

- during the selection step, the selected index(es) are the index(es) with the smallest root mean square error(s), wherein the root mean square error for each index verifies the following equation:

$$RMSE_{Index\_j} = \sqrt{\sum_{i=1}^{S} \frac{(y_i - \hat{y}_{i,j})^2}{S}}$$

where $y_i$ is the measured fitness of the $i^{th}$ sample protein,
$\hat{y}_{i,j}$ is the predicted fitness of the $i^{th}$ sample protein with the $j^{th}$ index, and
S the number of sample proteins;

- during the selection step, the selected index(es) are the index(es) with the coefficient(s) of determination nearest to 1, wherein the coefficient of determination for each index verifies the following equation:

$$R^2{}_{Index\_j} = \frac{(\sum_{i=1}^{S} (y_i - \overline{y})(\hat{y}_{i,j} - \overline{\hat{y}}))^2}{\sum_{i=1}^{S} (y_i - \overline{y})^2 \sum_{i=1}^{S} (\hat{y}_{i,j} - \overline{\hat{y}})^2}$$

where $y_i$ is the measured fitness of the $i^{th}$ sample protein,
$\hat{y}_{i,j}$ is the predicted fitness of the $i^{th}$ sample protein with the $j^{th}$ index,
S the number of sample proteins,
$\overline{y}$ is an average of the measured fitness for the S sample proteins, and
$\overline{\hat{y}}$ is an average of the predicted fitness for the S sample proteins;

- during the determining step, the elementary numerical sequences are concatenated according to a concatenation pattern for determining the extended numerical sequence, the reference extended numerical sequences having being obtained with the same concatenation pattern;
- the concatenation pattern defines, for each elementary numerical sequence from the succession of the elementary numerical sequences to be concatenated, the respective index and the applying or not of the Fourier Transform;
- the protein database includes several indexes classified into distinct categories, and the concatenation pattern includes indexes from at least two categories;
each category being preferably a family associated to a protein feature, such as a protein feature chosen from among the group consisting of: alpha & turn propensities, beta propensity, composition, hydrophobicity, physico-chemical property and other protein property; or
each category being preferably a cluster of index(es), the clusters being obtained according to statistical feature(s) of the indexes; and
- the comparison step comprises identifying, in the predetermined database of reference extended numerical sequences for different values of said fitness, the reference extended numerical sequence which is the closest according to a predetermined criterion to the determined extended numerical sequence, the predicted value of said fitness being then equal to the fitness value which is associated in said database with the identified reference extended numerical sequence.

The invention also relates to a computer program product including software instructions which, when implemented by a computer, implement a method as defined above.

The invention also relates to an electronic prediction system for predicting at least one fitness value of a protein, the prediction system including:

- a calculation module configured for calculating Q elementary numerical sequences, Q being an integer greater than or equal to 2, each elementary numerical sequence depending on a respective encoding of the amino acid sequence of the protein according to a protein database,
- a determination module configured for determining an extended numerical sequence by concatenating the Q elementary numerical sequences,
- a prediction module configured for, for each fitness:

    + comparing the determined extended numerical sequence with reference extended numerical sequences of a predetermined database, said database containing reference extended numerical sequences for different values of said fitness,
    + predicting a value of said fitness according to said comparison.

## Brief description of the drawings

The invention will be better understood upon reading of the following description, which is given solely by way of example and with reference to the appended drawings, in which:

- Figure 1 is a schematic view of an electronic prediction system for predicting at least one fitness value of a protein, the prediction system including a calculation module for calculating Q elementary numerical sequences, $Q \geq 2$, each one depending on a respective encoding of the amino acid sequence of the protein according to a protein database, a determination module for determining an extended numerical sequence by concatenating the Q elementary numerical sequences, and a prediction module configured for predicting at least one value of each fitness;
- Figure 2 is a schematic flow chart of a prediction method for predicting at least one fitness value of a protein, according to the invention;
- Figure 3 is a set of points illustrating predicted and measured values of the thermostability for a set of proteins of the cytochrome P450 family, each point being related to a respective protein with the ordinate corresponding to the predicted value and the abscissa corresponding to the measured value, with a prior art prediction method;
- Figure 4 is a view similar to that of Figure 3, with a prediction method according to the invention;
- Figure 5 is a view similar to that of Figure 3 illustrating predicted and measured values of the potency for a set of GLP2 mutants, with a prior art prediction method;
- Figure 6 is a view similar to that of Figure 5, with a prediction method according to the invention;
- Figures 7 and 8 are views similar to that of Figure 3 illustrating predicted and measured values of the enantioselectivity for a set of proteins of an epoxide hydrolase family, with a prior art prediction method;
- Figure 9 is a view similar to that of Figures 7 and 8, with a prediction method according to the invention;
- Figure 10 is a view similar to that of Figure 3 illustrating predicted and measured values of the binding affinity for a set of TNF mutants, with a prior art prediction method;
- Figure 11 is a view similar to that of Figure 10, with a prediction method according to the invention;
- Figures 12 to 14 are views similar to that of Figure 4, for other examples of respective encoding indexes used for encoding of the amino acid sequence of the protein into elementary numerical sequences;
- Figure 15 is a view similar to that of Figure 6, for another example of respective encoding indexes issued from different categories of index(es);
- Figure 16 is a view similar to that of Figure 11, for another example of respective encoding indexes issued from different clusters of index(es);
- Figure 17 is a view illustrating the different clusters of index(es) used in the example of Figure 16;
- Figure 18 is a view similar to that of Figure 11, for another example of respective encoding indexes;
- Figure 19, and respectively 20, are views similar to that of Figure 10, with a prior art prediction method, for a first encoding index, and respectively for a second encoding index;
- Figure 21 is a view similar to that of Figure 11, with a prediction method according to the invention, using the first and second encoding indexes of Figures 19 and 20;
- Figure 22 is a view similar to that of Figure 3, with a prediction method according to the invention, using an elementary protein spectrum for a given set of frequencies or harmonics; and
- Figures 23 and 24 are views similar to that of Figure 22, with a prediction method according to the invention, wherein a respective elementary numerical sequence is an elementary protein spectrum for a given set of frequency(ies) or harmonic(s).

## Detailed description of preferred embodiments

By "protein", as used herein, is meant at least 2 amino acids linked together by a peptide bond. The term "protein" includes proteins, oligopeptides, polypeptides and peptides. The peptidyl group may comprise naturally occurring amino acids and peptide bonds, or synthetic peptidomimetic structures, i.e. "analogs", such as peptoids. The amino acids may either be naturally occurring or non-naturally occurring. In preferred embodiments, a protein comprises at least 10 amino acids, but less amino acids can be managed.

The "fitness" of a protein refers to its adaptation to a criterion, such as catalytic efficacy, catalytic activity, kinetic constant, Km, Keq, binding affinity, thermostability, solubility, aggregation, potency, toxicity, allergenicity, immunogenicity, thermodynamic stability, flexibility, protein expression level and mRNA expression level. According to the invention, the "fitness" is also called "activity" and it will be considered in the following of the description that the fitness and the activity refer to the same feature.

The catalytic efficacy is usually expressed in $s^{-1}.M^{-1}$ and refers to the ratio kcat/Km.

The catalytic activity is usually expressed in $mol.s^{-1}$ and refers to the enzymatic activity level in enzyme catalysis.

The kinetic constant kcat is usually expressed in $s^{-1}$ and refers to the numerical parameter that quantifies the velocity of a reaction.

The Km is usually expressed in M and refers to the substrate concentration at which the velocity of reaction is half its maximum.

The Keq is usually expressed in ($M$, $M^{-1}$ or no unit) and quantity characterizing a chemical equilibrium in a chemical reaction.

The binding affinity is usually expressed in M and refers to the strength of interactions between proteins or proteins and ligand (peptide or small chemical molecule).

The thermostability is usually expressed in °C and usually refers to the measured activity $T_{50}$ defined as the temperature at which 50% of the protein is irreversibly denatured after incubation time of 10 minutes.

The solubility is usually expressed in mol/L and refers to the number of moles of a substance (the solute) that can be dissolved per liter of solution before the solution becomes saturated.

The aggregation is usually expressed using aggregation Index (from a simple absorption measurement at 280 nm and 340 nm) and refers to the biological phenomenon in which mis-folded protein aggregate (i.e., accumulate and clump together) either intra- or extracellularly.

The potency is usually expressed in M and refers to the measure of drug activity expressed in terms of the amount required to produce an effect of given intensity.

The toxicity is usually expressed in M and refers to the degree to which a substance (a toxin or poison) can harm humans or animals.

The allergenicity is usually expressed in Bioequivalent Allergy Unit per mL (BAU/mL) and refers to the capacity of an antigenic substance to produce immediate hypersensitivity (allergy).

The immunogenicity is usually expressed as the unit of the amount of antibody in a sample and refers to the ability of a particular substance, such as an antigen or epitope, to provoke an immune response in the body of a human or animal.

The stability is usually expressed as $\Delta\Delta G$ (kcal/mol-1) and refers to thermodynamic stability of a protein that unfolds and refolds rapidly, reversibly, and cooperatively.

The flexibility is usually expressed in A° and refers to protein disorder and conformational changes.

The protein expression level is usually expressed as a unit-less value, such as a percentage or a decimal value, and refers to the amount of production of proteins by cells.

The mRNA expression level is also usually expressed as a unit-less value, such as a percentage or a decimal value, and refers to the quantity of functional copies of mRNA in living cells.

The enantioselectivity refers to the preferential formation of one stereoisomer over another in a chemical reaction, or to the selectivity of a reaction towards one of a pair of enantiomers. The enantioselectivity is usually expressed by an E-value which is transformable in $\Delta\Delta G\ddagger$ (kcal/mol) by the relation $\Delta\Delta G \ddagger = -RT \ln (E)$.

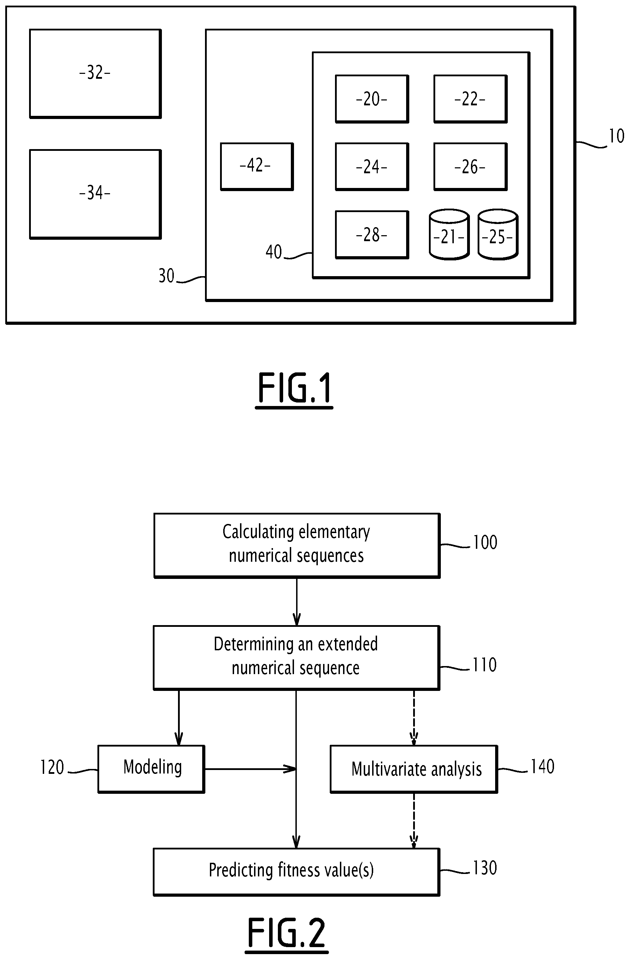

In Figure 1, an electronic prediction system 10 for predicting at least one fitness value of a protein includes a calculation module 20 configured for calculating Q elementary numerical sequences, Q being an integer greater than or equal to 2, each elementary numerical sequence depending on a respective encoding of the amino acid sequence of the protein according to a protein database 21.

The electronic prediction system 10 further includes a determination module 22 configured for determining an extended numerical sequence Ext_SEQ by concatenating the Q elementary numerical sequences.

In optional addition, the electronic prediction system 10 includes a modeling module 24 configured for predetermining a reference database 25, said reference database 25 containing reference extended numerical sequences for different values of said fitness.

The electronic prediction system 10 further includes a prediction module 26 configured for, for each fitness,

comparing the determined extended numerical sequence Ext_SEQ with reference extended numerical sequences of the reference database 25, and predicting a value of said fitness according to said comparison.

In optional addition, the electronic prediction system 10 includes a screening module 28 configured for analyzing the protein according to the determined extended numerical sequence Ext_SEQ, thereby for screening mutants' libraries, the analysis being for example a factorial discriminant analysis or a principal component analysis.

In the example of Figure 1, the electronic prediction system 10 includes a data processing unit 30, a display screen 32 and input means 34 for inputting data into the data processing unit 30.

The data processing unit 30 is, for example, made of a memory 40 and a processor 42 associated to the memory 40.

The display screen 32 and the input means 34 are known *per se.*

In the example of Figure 1, the calculation module 20, the determination module 22 and the prediction module 26, and in optional addition the modeling module 24 and/or the screening module 28, are for example each realized, i.e. implemented, as a software executable by the processor 42. The memory 40 of the processing unit 30 is adapted to store a calculation software configured for calculating Q elementary numerical sequences; a determination software configured for determining the extended numerical sequence Ext_SEQ by concatenating the Q elementary numerical sequences; and a prediction software configured for, for each fitness, comparing the determined extended numerical sequence Ext_SEQ with reference extended numerical sequences of the reference database 25 and predicting a value of said fitness according to said comparison. In optional addition, the memory 40 of the processing unit 30 is adapted to store a modeling software configured for predetermining the reference database 25 containing reference extended numerical sequences for different values of said fitness; and/or a screening software configured for analyzing the protein according to the determined extended numerical sequence Ext_SEQ, thereby for screening mutants' libraries. The processor 42 of the processing unit 30 is then configured to execute the calculation software, the determination software and the prediction software, and in optional addition the modeling software and/or the screening software.

As a variant not shown, the calculation module 20, the determination module 22 and the prediction module 26, and in optional addition the modeling module 24 and/or the screening module 28, are each in the form of a programmable logic component, such as a Field Programmable Gate Array or FPGA, or in the form of a dedicated integrated circuit, such as an Application Specific integrated Circuit or ASIC.

When the electronic prediction system 10 is in the form of one or more software programs, i.e. in the form of a computer program, it is also capable of being recorded on an computer-readable medium, not shown. The computer-readable medium is, for example, a medium capable of storing electronic instructions and being coupled to a bus of a computer system. For example, the readable medium is an optical disk, a magneto-optical disk, a ROM memory, a RAM memory, any type of non-volatile memory (for example EPROM, EEPROM, FLASH, NVRAM), a magnetic card or an optical card. A computer program with software instructions is then stored on the readable medium.

The calculation module 20 is configured for calculating several elementary numerical sequences, each elementary numerical sequence depending on a respective encoding of the amino acid sequence of the protein according to the protein database 21.

The calculation module 20 is for example adapted for encoding the amino acid sequence into respective elementary numerical sequence(s) according to the protein database 21, each elementary numerical sequence comprising a value $x_k$ for each amino acid of the sequence. The elementary numerical sequence is constituted of P value(s) $x_k$, with $0 \leq k \leq P-1$ and $P \geq 1$, k and P being integers.

In other words, encoding the amino acid sequence into a numerical sequence results in replacing each letter of amino acid in the amino acid sequence by a value.

The skilled person will notice that the amino acid sequence corresponds to the whole amino acid sequence of the protein or alternatively only to a partial amino acid sequence of the protein. According to this alternative, the partial amino acid sequence corresponds in other words to only one or several amino acid positions among the whole amino acid sequence of the protein.

The protein database 21 corresponds in a general manner to a set of relationship(s), wherein each relationship associates any numerical value to a given amino acid.

The protein database 21 is, for example, stored in the memory 40. Alternatively, the protein database 21 is stored in a remote memory, not shown, which is distinct from the memory 40.

The protein database 21 is for example the Amino Acid Index Database, also called AAIndex. Amino Acid Index Database is available from http://www.genome.jp/dbget-bin/www_bfind?aaindex (version Release 9.1, Aug 06). The AAIndex holds 566 indexes representing various physicochemical and biochemical properties for the 20 standard amino acids; and correlations between these indices are also listed in the AAIndex.

Alternatively, the protein database 21 contains predefined arbitrary numerical values, for example that range from 1 to $N_{AA}$, where $N_{AA}$ is the number of natural and/or non-natural amino acids in the protein database 21.

Further alternatively, the protein database 21 contains calculated numerical values for each amino acid, wherein these numerical values are calculated according to predefined calculation law or calculated randomly or pseudo-randomly.

Alternatively, or in addition, the protein database 21 contains numerical values for non-natural amino acids. The protein database 21 is for example based on the article "An index for characterization of natural and non-natural amino acids for peptidomimetics" from Liang, G., Liu, Y., Shi, B., Zhao, J., & Zheng, J., published in PloS one, 8(7), e67844, in 2013 and from the utilization of the application e-dragon, available from http://www.vcclab.org/lab/edragon which allows the calculation of physicochemical molecular descriptors from given molecules. The protein database 21 contains accordingly for example 615 non-natural amino acids with more of 1600 descriptors.

The protein database 21 includes at least one index of numerical values, each value being given for a respective amino acid. The protein database 21 includes preferably several indexes of numerical values.

The protein database 21 includes for example one or several indexes of biochemical or physico-chemical property values, each property value being given for a respective amino acid. Each index corresponds for example AAindex code, as it will be illustrated in the following in light of the respective examples. The chosen AAindex codes for encoding the amino acid sequence are for example: D Normalized frequency of extended structure, D Electron-ion interaction potential values, D SD of AA composition of total proteins, D pK-C or D Weights from the IFH scale.

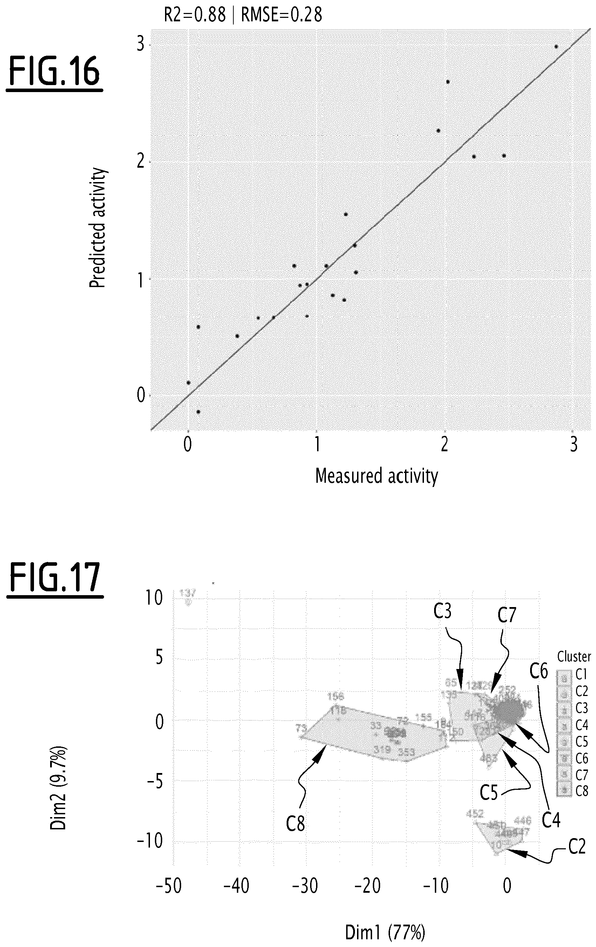

In optional addition, when the protein database 21 includes several indexes of numerical values, these several indexes are for example classified into distinct categories. According to a classification example, each category is a family associated to a protein feature, such as a protein feature chosen from among the group consisting of: alpha & turn propensities, beta propensity, composition, hydrophobicity, physicochemical property and other protein property. According to another classification example, each category is a cluster of index(es) which is obtained according to statistical feature(s) of the indexes. Figure 17 illustrates such a classification example with eight clusters C1 to C8.

For encoding the amino acid sequence, the calculation module 20 is then adapted to determine, for each amino acid, the numerical value for said amino acid according to the given index, each encoded value $x_k$ in the elementary numerical sequence being then equal to a respective numerical value.

In optional addition, when the protein database 21 includes several indexes of numerical values; the calculation module 20 is for example configured for selecting the best index based on a comparison of measured fitness values for sample proteins with predicted fitness values previously obtained for said sample proteins according to each index; and then for encoding the amino acid sequence using the selected index.

The selected index is, for example, the index with the smallest root mean square error, wherein the root mean square error for each index verifies the following equation:

$$RMSE_{Index\_j} = \sqrt{\sum_{i=1}^{S} \frac{(y_i - \hat{y}_{i,j})^2}{S}} \qquad (1)$$

where $y_i$ is the measured fitness of the $i^{th}$ sample protein,
$\hat{y}_{i,j}$ is the predicted fitness of the $i^{th}$ sample protein with the $j^{th}$ index, and
S the number of sample proteins.

Alternatively, the selected index is the index with the coefficient of determination nearest to 1, wherein the coefficient of determination for each index verifies the following equation:

$$R^2{}_{Index\_j} = \frac{(\sum_{i=1}^{S} (y_i - \overline{y})(\hat{y}_{i,j} - \overline{\hat{y}}))^2}{\sum_{i=1}^{S} (y_i - \overline{y})^2 \sum_{i=1}^{S} (\hat{y}_{i,j} - \overline{\hat{y}})^2} \qquad (2)$$

where $y_i$ is the measured fitness of the $i^{th}$ sample protein,
$\hat{y}_{i,j}$ is the predicted fitness of the $i^{th}$ sample protein with the $j^{th}$ index,
S the number of sample proteins,
$\overline{y}$ is an average of the measured fitness for the S sample proteins, and
$\overline{\hat{y}}$ is an average of the predicted fitness for the S sample proteins.

In optional addition, the calculation module 20 is further configured for normalizing the obtained elementary numerical sequence, for example by subtracting to each value $x_k$ of the elementary numerical sequence a mean $\overline{x}$ of the elementary numerical sequence values.

In other words, each normalized value, denoted $\tilde{x}_k$, verifies the following equation:

$$\tilde{x}_k = x_k - \overline{x} \tag{3}$$

The mean $\overline{x}$ is, for example, an arithmetic mean and satisfies:

$$\overline{x} = \frac{1}{P} \times \sum_{k=0}^{P-1} x_k \tag{4}$$

Alternatively, the mean $\overline{x}$ is a geometric mean, a harmonic mean or a quadratic mean.

In optional addition, the calculation module 20 is further configured for zero-padding the obtained elementary numerical sequence by adding M zeros at one end of said elementary numerical sequence, with M equal to (N - P) where N is a predetermined integer and P is the initial number of values in said elementary numerical sequence. N is therefore the total number of values in the elementary numerical sequence after zero-padding.

In optional addition, at least one elementary numerical sequence is an elementary protein spectrum, the elementary protein spectrum being obtained by applying a Fourier Transform, such as a Fast Fourier Transform, to an intermediate numerical sequence, the intermediate numerical sequence being obtained by a respective encoding of the amino acid sequence of the protein.

According to this optional addition, the calculation module 20 is configured for calculating the elementary protein spectrum according to the intermediate numerical sequence.

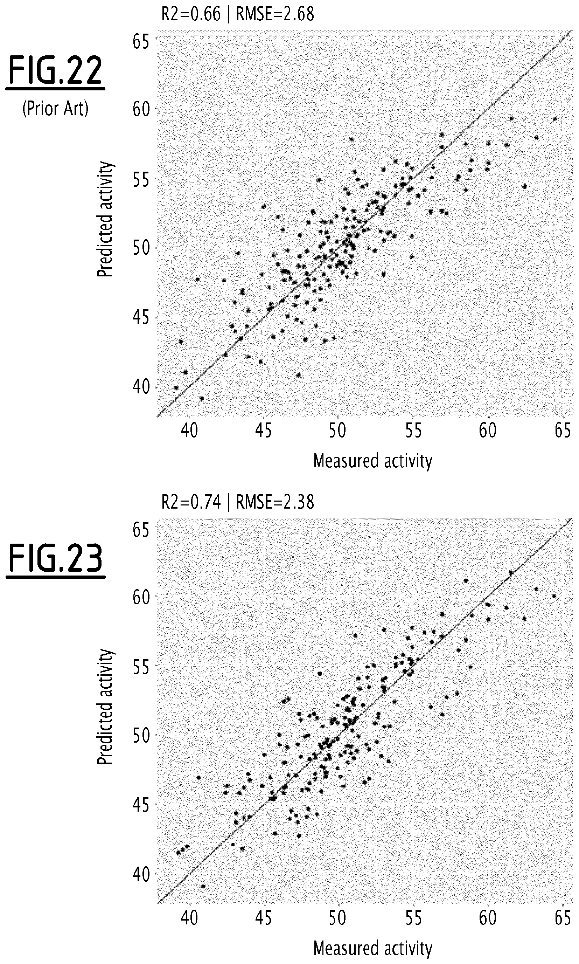

The calculated elementary protein spectrum includes at least one frequency value. The elementary protein spectrum is therefore calculated for the whole frequency spectrum or alternatively only according to a given set of frequency(ies) or harmonic(s) with one or several frequency values. This alternative with the elementary protein spectrum calculated only according to a given set of frequency(ies) or harmonic(s) will be further described later in view of the examples of Figures 22 to 24.

For determining the set of frequency(ies) or harmonic(s), i.e. for selecting frequency(ies) or harmonic(s), the calculation module 20 is for example configured for using a filter method or a wrapper method.

A filter method selects variables regardless of the model and is for example based only the correlation with the variable to predict. A filter method suppresses the least interesting variables. The other variables will be part of a classification or a regression model used to classify or to predict data. Such a filter method is for example carried out by correlating amplitude values at each harmonic with activity values (i.e. the values to be predicted), and then for selecting the harmonic(s) with the highest correlation. The correlation is for example evaluated according to the R2 and the set of frequency(ies) or harmonic(s) is then a given percentage frequency(ies) or harmonic(s) for which R2 is the highest.

A wrapper method evaluates subsets of variables which allows, unlike filter methods, to detect possible interactions between variables. Such a wrapper method is for example disclosed in the article from T. M. Phuong, Z. Lin et R. B. Altman: "Choosing SNPs using feature selection" in IEEE Computational Systems Bioinformatics Conference, pages 301-309, (2005).

The calculation module 20 is configured for calculating the elementary protein spectrum $f_j$, preferably by applying a Fourier Transform, such as a Fast Fourier Transform, to the obtained intermediate numerical sequence.

Each elementary protein spectrum $f_j$ therefore verifies, for example, the following equation:

$$f_j = \sum_{k=0}^{P-1} x_k \cdot exp\left(\frac{-2i\pi}{P} \cdot j \cdot k\right) \tag{5}$$

where j is an index-number of the elementary protein spectrum $f_j$; and
i defines the imaginary number such that $i^2 = -1$.

In optional addition, when the intermediate numerical sequence is normalized, the calculation module 20 is further configured for performing the elementary protein spectrum calculation on the normalized intermediate numerical sequence.

In other words, in this case, each elementary protein spectrum $f_j$ therefore verifies, for example, the following equation:

$$f_j = \sum_{k=0}^{P-1} \tilde{x}_k \cdot exp\left(\frac{-2i\pi}{P} \cdot j \cdot k\right) \quad (6)$$

In optional addition, when zero-padding is performed on the intermediate numerical sequence, the calculation module 20 is further configured for calculating the elementary protein spectrum $f_j$ on the intermediate numerical sequence obtained further to zero-padding.

In other words, in this case, each elementary protein spectrum $f_j$ therefore verifies, for example, the following equation:

$$f_j = \sum_{k=0}^{N-1} x_k \cdot exp\left(\frac{-2i\pi}{N} \cdot j \cdot k\right) \quad (7)$$

In optional addition, when both normalization and zero-padding are performed on the intermediate numerical sequence, the calculation module 20 is further configured for calculating the elementary protein spectrum $f_j$ on the normalized intermediate numerical sequence obtained further to zero-padding.

In other words, in this case, each elementary protein spectrum $f_j$ therefore verifies, for example, the following equation:

$$f_j = \sum_{k=0}^{N-1} \tilde{x}_k \cdot exp\left(\frac{-2i\pi}{N} \cdot j \cdot k\right) \quad (8)$$

The determination module 22 is configured for determining the extended numerical sequence Ext_SEQ by concatenating the Q elementary numerical sequences.

In the extended numerical sequence Ext_SEQ determined by the determination module 22, all the elementary numerical sequences are distinct from each other.

For example, among a pair of elementary numerical sequences, one differs from the other further to the applying of the Fourier Transform for only one elementary numerical sequence of the pair. In the following of the description, an elementary numerical sequence obtained further to the applying of the Fourier Transform is denoted FFT_Seq for a single encoding index, or FFT_Seq$_{j1}$, FFT_Seq$_{j2}$, when several encoding indexes j1, j2 are taken into consideration. Conversely, an elementary numerical sequence obtained without applying the Fourier Transform is denoted noFFT_Seq for a single encoding index, or noFFT_Seq$_{j1}$, noFFT_Seq$_{j2}$, when several encoding indexes j1, j2 are taken into consideration.

In addition, or alternatively, among a pair of elementary numerical sequences, one differs for example from the other further to a different index from the one to the other elementary numerical sequence of the pair.

As an example, if the amino acid sequence of the protein is encoded according to only one encoding index, the determination module 22 is configured for determining the extended numerical sequence Ext_SEQ according to the following formulation:

$$\text{Ext\_SEQ = noFFT\_Seq--FFT\_Seq} \quad (9)$$

where the symbol "--" between the two elementary numerical sequences noFFT Seq and FFT_Seq represents the concatenation of these two elementary numerical sequences.

According to another example, if the amino acid sequence of the protein is encoded according to two distinct encoding indexes j1 and j2, the determination module 22 is configured for determining the extended numerical sequence Ext_SEQ according to the following possible alternative formulations:

$$\text{Ext\_SEQ = noFFT\_Seq}_{j1}\text{--noFFT\_Seq}_{j2} \quad (10)$$

$$\text{Ext\_SEQ = FFT\_Seq}_{j1}\text{--noFFT\_Seq}_{j2} \quad (11)$$

$$\text{Ext\_SEQ = noFFT\_Seq}_{j1}\text{--FFT\_Seq}_{j2} \quad (12)$$

$$\text{Ext\_SEQ = FFT\_Seq}_{j1}\text{--FFT\_Seq}_{j2} \quad (13)$$

The skilled person will naturally derive, from the aforementioned formulations, the possible alternative formulations of the extended numerical sequence Ext_SEQ in the case wherein the amino acid sequence of the protein is encoded according a number Nb_Index of distinct encoding indexes j1, j2, ..., $j_{Nb\_Index}$ which is strictly greater than 2.

It should be noted that even if all the elementary numerical sequences are distinct from each other, all the elementary numerical sequences preferably correspond, for a given extended numerical sequence Ext_SEQ, to the same amino acid sequence of the protein. All the elementary numerical sequences therefore depend, for a given extended numerical sequence Ext_SEQ, on a single amino acid sequence of the protein. Indeed, the electronic prediction system 10 according to the invention aims at better predicting fitness value(s) of said amino acid sequence of the protein. In other words, the elementary numerical sequences differ from one another through the encoding index and/or through applying or not of the Fourier Transform.

The above formulations each represent a concatenation pattern for concatenating the elementary numerical sequences into the determined extended numerical sequence Ext_SEQ.

In other words, the concatenation pattern defines, for each elementary numerical sequence from the succession of the elementary numerical sequences to be concatenated, the respective index and the applying or not of the Fourier Transform.

The determination module 22 is configured for concatenating the Q elementary numerical sequences into the extended numerical sequence Ext_SEQ according to the concatenation pattern. The concatenation pattern is preferably a predefined concatenation pattern.

In optional addition, when the protein database 21 includes several indexes classified into distinct categories, the concatenation pattern includes for example indexes from at least two distinct categories.

In optional addition, when the protein database 21 includes several indexes, the best indexes are for example selected by determining at first the best index j1 as above explained, and then by identifying the second best j2 in the remaining set of indexes which corresponds to the initial set of indexes less the best index (determined at first); and so on.

As an example, with the AAIndex including 566 indexes, the 566 indexes are tested one by one. A ranking of the 566 indexes of the protein database 21 is done according the cvRMSE value during a cross-validation procedure. The best index j1 is the one that gives the lowest cvRMSE. Then, the second-best index j2 is identified by testing successively, once again all the (566-1) indexes. At the end of the process, the second index j2 is chosen according to the lowest value of the cvRMSE as obtained using a LOOCV. And so on for a third-best index j3.

The modeling module 24 is adapted for predetermining the protein spectra database 25, also called model, according to learning data and learning extended numerical sequences. The learning extended numerical sequences correspond to the learning data and the learning data are each related to a given fitness, and preferably for different values of said fitness.

The modeling module 24 is further configured for obtaining the reference extended numerical sequences with the same concatenation pattern as the one used by the determination module 22 for concatenating the Q elementary numerical sequences into the extended numerical sequence Ext_SEQ.

The reference database 25 contains reference extended numerical sequences for different values of said fitness. Preferably, at least 10 extended numerical sequences and 10 different fitness are used to build the reference database 25. Of course, the higher are the number of reference extended numerical sequences and related protein fitness; the better will be the results in terms of prediction of fitness.

The prediction module 26 is adapted, for each fitness, for comparing the determined extended numerical sequence Ext_SEQ with reference extended numerical sequences of the reference database 25 and predicting a value of said fitness according to said comparison.

The prediction module 26 is preferably further configured for identifying, in the predetermined database 25 of reference extended numerical sequences for different values of said fitness, the reference extended numerical sequence which is the closest according to a predetermined criterion to the determined extended numerical sequence Ext_SEQ, the predicted value of said fitness being then equal to the fitness value which is associated in said database with the identified reference extended numerical sequence.

The predetermined criterion is, for example, the minimum difference between the determined extended numerical sequence Ext_SEQ and the reference extended numerical sequences contained in the reference database 25. Alternatively, the predetermined criterion is the correlation coefficient R or determination coefficient $R^2$ between the determined extended numerical sequence Ext_SEQ and the reference extended numerical sequences contained in the reference database 25.

Alternatively, the prediction module 26 is configured for computing the predicted value of the fitness using an Artificial Neural Network (ANN), with the input variable being the determined extended numerical sequence Ext_SEQ and the output variable being the predicted value of the fitness. According to this alternative, the Artificial Neural Network is previously trained on the reference extended numerical sequences of the reference database 25 which have the same concatenation pattern as the one used for determining the extended numerical sequence Ext_SEQ.

In addition, in an optional manner, the prediction module 26 allows obtaining a screening of mutants' libraries.

In addition, in an optional manner, the screening module 28 is adapted for analyzing proteins according to the determined extended numerical sequence Ext_SEQ, and for classifying protein sequences according to their respective extended numerical sequence Ext_SEQ using mathematical treatments, such as a factorial discriminant analysis or a principal component analysis followed for example by a k-means. The classification can be done for example to identify if in a family of protein spectra different groups exist: groups with high, intermediate and low fitness; a group with an expression of fitness and a group with no expression of fitness, as examples.

The operation of the electronic prediction system 10 according to the invention will now be described in view of Figure 2 representing a flow chart of the method for predicting at least one fitness value of a protein.

In an initial step 100, the calculation module 20 calculates several elementary numerical sequences, each elementary numerical sequence depending on a respective encoding of the amino acid sequence of the protein according to the protein database 21.

The calculation module 20 encodes the amino acid sequence into respective elementary numerical sequence(s) according to the protein database 21 by determining, for each amino acid, the numerical value for said amino acid in the given index, for example in the given AAindex code, and then issues an encoded value $x_k$ which is equal to said numerical value.

In addition, when the protein database 51 optionally includes several indexes of numerical values; the encoding module 50 further selects the best index based as described above; and then encodes the amino acid sequence using the selected index. The best index is, for example, selected using equation (1) or equation (2).

Alternatively, or in addition, when the protein database 51 optionally includes several indexes of numerical values; the encoding module 50 uses several indexes for encoding several respective elementary numerical sequences.

In optional addition, the calculation module 20 optionally normalizes each obtained elementary numerical sequence, for example by subtracting to each value $x_k$ of the numerical sequence a mean $\overline{x}$ of the numerical sequence values according to equation (3).

In optional addition, the calculation module 20 optionally performs zero-padding on the obtained elementary numerical sequence by adding M zeros at one end of said elementary numerical sequence.

In optional addition, at least one elementary numerical sequence is an elementary protein spectrum, and the calculation module 20 applies accordingly a Fourier Transform, such as a Fast Fourier Transform, to an intermediate numerical sequence obtained by a respective encoding of the amino acid sequence of the protein, in order to obtain the corresponding elementary protein spectrum. The elementary protein spectra $f_j$ are preferably calculated by using a Fourier Transform, such as a Fast Fourier Transform, for example according to an equation among the equations (5) to (8) depending on an optional normalization and/or zero-padding.

In the next step 110, the determination module determines the extended numerical sequence Ext_SEQ by concatenating the Q elementary numerical sequences, all the elementary numerical sequences being distinct from each other.

For example, among a pair of elementary numerical sequences, one differs from the other further to the applying of the Fourier Transform for only one elementary numerical sequence of the pair and/or further to a different index from the one to the other elementary numerical sequence of the pair.

The determination module 22 for example determines the extended numerical sequence Ext_SEQ according to the formulation (9) in the case of a single encoding index, or according to any one formulations (10) to (13) in the case of two distinct encoding indexes, or according to similar formulations with at least elementary numerical sequences in the case of more than two distinct encoding indexes.

At the end of the determining step 110, the determination module 22 delivers learning data and learning extended numerical sequences to the modeling module 24.

Then, the modeling module 24 determines, in step 120, the reference database 25 according to learning data and learning extended numerical sequences obtained at the end of the determining step 110.

During the modeling step 120, the modeling module 24 evaluates multiple encoding indexes to find the best for the construction of models. For example, the modeling module 24 therefore uses an initial dataset, also called training dataset, to construct a predictive model for each encoding index. For each model, the modeling module 24 calculates the value of the performance parameters in two stages. A first stage is a standard cross validation. A second stage is a modeling integrating the full set in the learning step. The performances from the two stages are analyzed in order to evaluate and to check the robustness and the validity of the model.

In the first stage, i.e. the cross-validation stage, the initial dataset is split into k equal portions. The number k varies according to the size of the initial dataset. The modeling module 24 uses a low k value if the dataset size is high and conversely a high k value in the opposite situation. The modeling module 24 uses k-1 portions as the learning dataset and the remaining one as the test dataset. This is repeated k more times until each portion is used as the testing dataset once. The cross-validation allows to avoid potential overfitting problem and to optimize some modeling parameters. The cross-validation is for example the Leave-One-Out Cross-Validation (LOOCV), where k is equal to the number Q of elementary numerical sequences.

In the second stage, the full set stage, the whole initial dataset is used as a learning dataset and a test dataset is tested with the optimized parameters from the first stage. In this second stage, the modeling module 24 checks the accuracy of the predictions for learned sequences.

At the end of the modeling step 120, the modeling module 24 selects and stores the reference database 25 a set of accurate models and their associated encoding indexes.

In step 130, for each fitness, the prediction module 26 compares the determined extended numerical sequence Ext_SEQ with reference extended numerical sequences of the reference database 25 and predicts a value of said fitness according to said comparison.

More precisely, the prediction module 26 identifies, in the reference database 25, the reference extended numerical sequence which is the closest according to a predetermined criterion to the determined extended numerical sequence Ext_SEQ, the predicted value of said fitness being then equal to the fitness value which is associated in said database with the identified reference extended numerical sequence.

Alternatively, the prediction module 26 computes the predicted value of the fitness using the Artificial Neural Network (ANN), with the input variable being the determined extended numerical sequence Ext_SEQ and the output variable being the predicted value of the fitness. According to this alternative, the Artificial Neural Network is previously trained on the reference extended numerical sequences of the reference database 25 which have the same concatenation pattern as the one used for determining the extended numerical sequence Ext_SEQ.

Finally, and optionally, the screening module 28 analyzes, in step 140, proteins according to the determined extended numerical sequence Ext_SEQ and classifies protein sequences according to their respective extended numerical sequence Ext_SEQ using mathematical treatments, such as a factorial discriminant analysis or a principal component analysis followed for example by a k-means.

It therefore allows obtaining a better screening of mutants' libraries. This step is also called multivariate analysis step.

It should be noted that the analysis step 140 directly follows the determining step 120 and that in addition the predicting step 130 may be performed after the analysis step 140 for predicting fitness values for some or all of the classified proteins.

## Examples

The invention will be further illustrated in view of the following examples.

In these examples, four datasets have been used: a cytochrome P450 dataset, a GLP2 dataset, an epoxide hydrolase dataset and a TNF dataset.

The versatile cytochrome P450 family of heme-containing redox enzymes hydroxylates a wide range of substrates to generate products of significant medical and industrial importance.

3 parental cytochrome P450, i.e. CYP102A1 (SEQ ID NO: 1), CYP102A2 (SEQ ID NO: 2) and CYP102A3 (SEQ ID NO: 3), were used to generate 184 chimeric sequences of cytochrome P450. For each variant, the thermostability was analyzed by the measurement of the temperature $T_{50}$, at which 50% of the protein irreversibly denatured after incubation for 10 min. This dataset was disclosed in the article "A diverse family of thermostable cytochrome P450s created by recombination of stabilizing fragments" from Li, Y., Drummond, D. A., Sawayama, A. M., Snow, C. D., Bloom, J. D. & Arnold, F. H., published in 2007 in Nature biotechnology, 25(9), 1051.

Table 2: cytochrome P450 learning set

| Chimera | Thermostability (°C) |
|---|---|
| 11111111 | 54.9 |
| 22222222 | 43.6 |
| 33333333 | 49.1 |
| 32233232 | 39.8 |
| 32313233 | 52.9 |
| 21133233 | 48.8 |
| 31312113 | 45 |
| 21332223 | 48.3 |
| 21312323 | 61.5 |
| 22312322 | 54.6 |

(continued)

| Chimera | Thermostability (°C) |
|---|---|
| 21212112 | 51.2 |
| 23133121 | 47.3 |
| 11312233 | 51.6 |
| 21133312 | 45.4 |
| 21133313 | 50.8 |
| 11332233 | 43.3 |
| 31212332 | 53.4 |
| 12211232 | 49.1 |
| 31312133 | 52.6 |
| 12232332 | 39.2 |
| 22133232 | 47.9 |
| 22233221 | 46.8 |
| 23113323 | 51 |
| 11332212 | 47.8 |
| 32332231 | 49.4 |
| 22132331 | 53.3 |
| 23313111 | 56.9 |
| 23112323 | 46 |
| 11113311 | 51.2 |
| 21232233 | 50.6 |
| 12332233 | 47.1 |
| 23333311 | 45.7 |
| 32132233 | 42.9 |
| 22331123 | 47.9 |
| 12212332 | 48.4 |
| 31212323 | 48.7 |
| 32312322 | 49.1 |
| 32312231 | 52.6 |
| 21232332 | 49.3 |
| 31331331 | 47.3 |
| 21132222 | 45.6 |
| 21212333 | 63.2 |
| 21231233 | 50.6 |
| 22212322 | 50.7 |
| 21112122 | 50.3 |
| 22111223 | 51.3 |
| 23233212 | 39.5 |
| 31312212 | 48.9 |

(continued)

| Chimera | Thermostability (°C) |
|---------|----------------------|
| 32211323 | 46.6 |
| 21213231 | 54.9 |
| 21332312 | 52.9 |
| 22332211 | 53 |
| 22113323 | 53.8 |
| 22113332 | 48.7 |
| 22213132 | 52 |
| 31213332 | 50.8 |
| 22113211 | 51.1 |
| 22313323 | 60 |
| 32333233 | 47.2 |
| 22331223 | 51.7 |
| 23333233 | 51 |
| 22333332 | 49 |
| 23332331 | 48 |
| 21233132 | 42.4 |
| 13333211 | 45.7 |
| 22232331 | 50.5 |
| 22313233 | 58.5 |
| 31311233 | 56.9 |
| 21132321 | 49.3 |
| 32212231 | 47.4 |
| 23212212 | 48 |
| 22113223 | 49.9 |
| 22233211 | 46.3 |
| 23213311 | 49.5 |
| 31212321 | 44.9 |
| 23112233 | 51 |
| 32332323 | 48.5 |
| 22112223 | 52.8 |
| 32313231 | 52.5 |
| 32132232 | 42.5 |
| 22232233 | 49.6 |
| 22232322 | 45.4 |
| 22333211 | 50.7 |
| 22332223 | 52.4 |
| 23213212 | 49 |
| 23333213 | 50.1 |

(continued)

| Chimera | Thermostability (°C) |
|---------|---------------------|
| 31312233 | 57.9 |
| 22232333 | 53.7 |
| 31333233 | 46.5 |
| 22213212 | 50.5 |
| 22132212 | 46.6 |
| 21332233 | 58.9 |
| 23333131 | 50.5 |
| 31312332 | 54.9 |
| 21333221 | 51.3 |
| 22333223 | 49.9 |
| 21111333 | 62.4 |
| 12212212 | 44.8 |
| 11313233 | 48.3 |
| 32113232 | 47.9 |
| 21113322 | 50.4 |
| 31313232 | 51.9 |
| 23213333 | 56.1 |
| 21333233 | 54.2 |
| 22233212 | 44 |
| 21313112 | 54.8 |
| 31213233 | 50.6 |
| 22132113 | 40.6 |
| 31112333 | 55.7 |
| 31212331 | 51.8 |
| 22232222 | 47.5 |
| 23332221 | 46.4 |
| 21332131 | 58.5 |
| 23231233 | 45.5 |
| 22111332 | 50.9 |
| 23312121 | 49.3 |
| 22332222 | 50.3 |
| 23312323 | 53.8 |
| 21131121 | 53 |
| 32212232 | 48.8 |
| 22112323 | 55.3 |
| 21232232 | 49.5 |
| 11212333 | 50.4 |
| 31212232 | 51 |

(continued)

| Chimera | Thermostability (°C) |
|---|---|
| 23213211 | 47.4 |
| 11331312 | 43.5 |
| 23331233 | 50.9 |
| 22133323 | 49.4 |
| 33333233 | 46.3 |
| 22233323 | 48.4 |
| 32232131 | 43.9 |
| 31312323 | 52.3 |
| 21313313 | 64.4 |
| 22333231 | 53.1 |
| 22232123 | 43.1 |
| 21132323 | 50.1 |
| 23332231 | 51.4 |
| 12112333 | 50.9 |
| 22133212 | 47.2 |
| 31113131 | 54.9 |
| 23313333 | 61.2 |
| 21113133 | 51.9 |
| 21111323 | 54.4 |
| 22212123 | 47.7 |
| 12211333 | 50.6 |
| 23113112 | 46.3 |
| 21313122 | 50.5 |
| 23112333 | 54.3 |
| 12213212 | 44 |
| 23132233 | 43.6 |
| 21313311 | 56.9 |
| 21332231 | 60 |
| 23133233 | 43.1 |
| 21132212 | 48.8 |
| 23313233 | 56.3 |
| 21332322 | 48.8 |
| 22132231 | 53 |
| 21113312 | 53 |
| 22312223 | 56.2 |
| 23332223 | 46.7 |
| 32212323 | 48.4 |
| 21212111 | 57.2 |

(continued)

| Chimera | Thermostability (°C) |
|---|---|
| 31212212 | 47.1 |
| 22232121 | 49.7 |
| 21232212 | 47.8 |
| 21333223 | 49.1 |
| 23213232 | 48.5 |
| 22113232 | 51.1 |
| 11331333 | 46.3 |
| 22333321 | 49.2 |
| 21232321 | 46 |
| 31332233 | 49.9 |
| 21133232 | 46.4 |
| 22112211 | 54.7 |
| 21333333 | 58 |
| 22213223 | 50.8 |
| 21332112 | 50.4 |
| 21331332 | 52 |
| 11313333 | 53.8 |
| 32311323 | 52 |
| 23132231 | 48 |
| 12232232 | 40.9 |
| 21212231 | 59.9 |
| 33312333 | 54.7 |
| 22313232 | 58.8 |
| 22312111 | 53 |
| 32212233 | 49.9 |

The GLP2 dataset involves the potency of 31 alanine variants of the Glucagon like peptide-2 (GLP-2) with respect to the activation of its receptor. GLP-2 (SEQ ID NO: 4) is a short 33 residues peptide whose increase in activity has direct implication in the control of epithelial growth in the intestine. The value for the corresponding receptor activation for the 31 alanine variants of GLP-2 is defined as the fold increase over basal cAMP production and are ranged from 0.7 to 10.4.

Table 3: GLP2 learning set

| Variant | fold-increase in cAMP |
|---|---|
| A2G | 10.00 |
| H1A | 0.78 |
| D3A | 1.02 |
| G4A | 2.87 |
| S5A | 4.00 |
| F6A | 1.67 |

(continued)

| Variant | fold-increase in cAMP |
|---------|----------------------|
| S7A | 7.59 |
| D8A | 7.53 |
| E9A | 3.76 |
| M10A | 0.78 |
| N11A | 7.65 |
| T12A | 3.11 |
| I13A | 2.45 |
| L14A | 2.03 |
| D15A | 4.54 |
| N16A | 5.92 |
| L17A | 1.02 |
| R20A | 3.94 |
| D21A | 4.42 |
| F22A | 1.61 |
| I23A | 2.57 |
| N24A | 10.00 |
| W25A | 1.37 |
| L26A | 2.33 |
| I27A | 5.02 |
| Q28A | 5.80 |
| T29A | 4.78 |
| K30A | 0.84 |
| I31A | 4.42 |
| T32A | 0.84 |
| D33A | 3.41 |

The epoxide hydrolase dataset, disclosed in the article from Reetz, M. T., & Sanchis, J. (2008) "Constructing and analyzing the fitness landscape of an experimental evolutionary process". ChemBioChem, 9(14), 2260-2267, is a collection of 37 mutants and one WT sequences from Aspergillus niger (WT sequence corresponds to SEQ ID NO: 5) and their enantioselectivity. This enzyme is known for the hydrolysis of glycidyl phenyl ether. The epoxide hydrolase allows the synthesis of important intermediates for the synthesis of beta-blockers, commonly used pharmaceutical drugs in hypertension treatment (Iakovou, K., Kazanis, M., Vavayannis, A., Bruni, G., Romeo, M. R., Massarelli, P., ... & Mori, T. (1999). "Synthesis of oxypropanolamine derivatives of 3, 4-dihydro-2H-1, 4-benzoxazine, β-adrenergic affinity, inotropic, chronotropic and coronary vasodilating activities". European journal of medicinal chemistry, 34(11), 903-917). The study of Reetz et al identifies epoxide mutants with an improved selectivity toward the enantiomer S.

Table 4: epoxide hydrolase learning set

| Variant | E-value | $\Delta\Delta G$ values (kcal/mol) |
|---------|---------|-----------------------------------|
| WT | 4 | -0.85 |
| L215F | 12 | -1.5 |
| A217N | 7 | -1.17 |

(continued)

| Variant | E-value | ΔΔG values (kcal/mol) |
|---|---|---|
| R219S | 4 | -0.85 |
| L249Y | 4 | -0.85 |
| T317W | 12 | -1.5 |
| T318V | 4 | -0.85 |
| M329P | 6 | -1.08 |
| L330Y | 4 | -0.85 |
| C350V | 5 | -0.97 |
| L215F_A217N_R219S | 16.29 | -1.68 |
| M329P_L330Y | 4.24 | -0.87 |
| T317W_T318V | 16.29 | -1.68 |
| L215F_A217N_R219S_M329P_L330Y | 21.25 | -1.84 |
| L215F_A217N_R219S_C350V | 16.02 | -1.67 |
| L215F_A217N_R219S_T317W_T318V | 38.01 | -2.19 |
| L215F_A217N_R219S_L249Y | 24.68 | -1.93 |
| M329P_L330Y_C350V | 4.46 | -0.9 |
| T317W_T318V_M329P_L330Y | 8.67 | -1.3 |
| L249Y_M329P_L330Y | 5.09 | -0.98 |
| T317W_T318V_C350V | 17.7 | -1.73 |
| L249Y_C350V | 4.39 | -0.89 |
| L249Y_T317W_T318V | 22.71 | -1.88 |
| L215F_A217N_R219S_M329P_L330Y_C350V | 24.27 | -1.92 |
| L215F_A217N_R219S_T317W_T318V_M329P_L330Y | 35.56 | -2.15 |
| L215F_A217N_R219S_L249Y_M329P_L330Y | 25.94 | -1.96 |
| L215F_A217N_R219S_T317W_T318V_C350V | 54.77 | -2.41 |
| L215F_A217N_R219S_L249Y_C350V | 21.61 | -1.85 |
| L215F_A217N_R219S_L249Y_T317W_T318V | 51.25 | -2.37 |
| T317W_T318V_M329P_L330Y_C350V | 12.28 | -1.51 |
| L249Y_M329P_L330Y_C350V | 4.61 | -0.92 |
| L249Y_T317W_T318V_M329P_L330Y | 18.3 | -1.75 |
| L249Y_T317W_T318V_C350V | 18 | -1.74 |
| L215F_A217N_R219S_L249Y_M329P_L330Y_C350V | 71.45 | -2.57 |
| L215F_A217N_R219S_T317W_T318V_M329P_L330Y_C350V | 32.19 | -2.09 |
| L215F_A217N_R219S_L249Y_T317W_T318V_M329P_L330Y | 47.17 | -2.32 |
| L215F_A217N_R219S_L249Y_T317W_T318V_C350V | 93.2 | -2.73 |
| L215F_A217N_R219S_L249Y_T317W_T318V_M329P_L330Y_C350V | 117.6 | -2.87 |

The TNF dataset, disclosed in the article from Mukai Y et al. (J Mol Biol. 2009 Jan 30;385(4):1221-9) *"Structure-function relationship of tumor necrosis factor (TNF) and its receptor interaction based on 3D structural analysis of a fully active TNFR1-selective TNF mutant",* is a collection of 20 mutants and one WT Tumour Necrosis Factor (TNF) sequences (WT sequence corresponds to SEQ ID NO: 6) . TNF is an important cytokine that suppresses carcinogenesis and excludes infectious pathogens to maintain homeostasis. The relative affinity (%Kd) of TNF to its two receptors, TNFR1 and TNFR2 is computed as a single ratio of $\log_{10}(R1/R2)$ which ranges from 0 to 2.87, where R1 and R2 are affinities of TNF to TNFR1 and TNFR2 respectively as measured by $IC_{50}$ assays in ng/ml.

Table 5: TNF learning set

| Variant | relative binding affinities |
|---|---|
| WT_157aa | 0 |
| K11M_K65S_K90P_K98R_K112N_K128P | 0.079 |
| L29I | 0.079 |
| A84T_V85H_S86K_Q88P_T89Q | 0.544 |
| A84S_V85K_S86T_Q88S_T89H | 0.663 |
| L29Q_H32W | 0.826 |
| L29K_R31A_R32G_E146S_S147T | 0.924 |
| A84S_V85T_S86N_Q88N_T89G | 0.869 |
| A84S_V85S_S86H_Q88R_T89F | 1.079 |
| A84S_V85P_S86L_Q88P_T89K | 1.217 |
| A84T_V85S_S86A_Q88G_T89P | 1.23 |
| A84T_V85T_S86A_Q88S_T89G | 1.31 |
| A145R_E146T_S147D | 1.301 |
| A145K_E146D_S147T | 2.87 |
| A145R_E146E_S147T | 2.228 |
| A145A_E146D_S147D | 1.949 |
| A145A_E146N_S147D | 2.462 |
| L29T_R31G_R32Y | 0.38 |
| L29T_R31K_R32Y | 1.127 |
| L29T_R32F_E146T | 2.026 |
| A84S_V85K_S86T_Q88T_T89H | 0.924 |

The encoding indexes used in the following examples are listed in Table 6 hereinafter which defines correspondence between the index number and the name of the index in the AAindex database, while indicating the dataset for which the corresponding encoding index was used in the following examples.

Table 6

| Index number | Index name | Dataset |
|---|---|---|
| 39 | Normalized frequency of beta-sheet (Chou-Fasman, 1978b) | Cytochrome P450 |
| 226 | Normalized frequency of beta-sheet from CF (Palau et al., 1981) | Cytochrome P450 |
| 300 | Average relative fractional occurrence in A0(i) (Rackovsky-Scheraga, 1982) | Cytochrome P450 |
| 343 | Information measure for extended (Robson-Suzuki, 1976) | Cytochrome P450 |
| 450 | Hydropathy scale based on self-information values in the two-state model (25% accessibility) (Naderi-Manesh et al., 2001) | Cytochrome P450 |
| 14 | Transfer free energy to surface (Bull-Breese, 1974) | Epoxide hydrolase |
| 161 | Normalized frequency of beta-sheet, with weights (Levitt, 1978) | Epoxide hydrolase |

(continued)

| Index number | Index name | Dataset |
|---|---|---|
| 178 | Retention coefficient in HPLC, pH7.4 (Meek, 1980) | Epoxide hydrolase |
| 232 | Normalized frequency of beta-sheet in all-beta class (Palau et al., 1981) | Epoxide hydrolase |
| 254 | Relative frequency in beta-sheet (Prabhakaran, 1990) | Epoxide hydrolase |
| 303 | Average relative fractional occurrence in EL(i) (Rackovsky-Scheraga, 1982) | Epoxide hydrolase |
| 508 | Linker propensity from helical (annotated by DSSP) dataset (George-Heringa, 2003) | Epoxide hydrolase |
| 516 | Hydrostatic pressure asymmetry index, PAI (Di Giulio, 2005) | Epoxide hydrolase |
| 44 | Normalized frequency of C-terminal non helical region (Chou-Fasman, 1978b) | GLP2 |
| 193 | AA composition of mt-proteins from animal (Nakashima et al., 1990) | GLP2 |
| 233 | Normalized frequency of beta-sheet in alpha+beta class (Palau et al., 1981) | GLP2 |
| 341 | Information measure for middle helix (Robson-Suzuki, 1976) | GLP2 |
| 350 | Information measure for coil (Robson-Suzuki, 1976) | GLP2 |
| 440 | Distribution of amino acid residues in the 18 non-redundant families of thermophilic proteins (Kumar et al., 2000) | GLP2 |
| 449 | Hydropathy scale based on self-information values in the two-state model (20% accessibility) (Naderi-Manesh et al., 2001) | GLP2 |
| 203 | AA composition of CYT2 of single-spanning proteins (Nakashima-Nishikawa, 1992) | TNF |
| 297 | Average reduced distance for C-alpha (Rackovsky-Scheraga, 1977) | TNF |
| 486 | Electron-ion interaction potential values (Cosic, 1994) | TNF |
| 504 | Linker propensity from 3-linker dataset (George-Heringa, 2003) | TNF |
| 523 | Apparent partition energies calculated from Chothia index (Guy, 1985) | TNF |

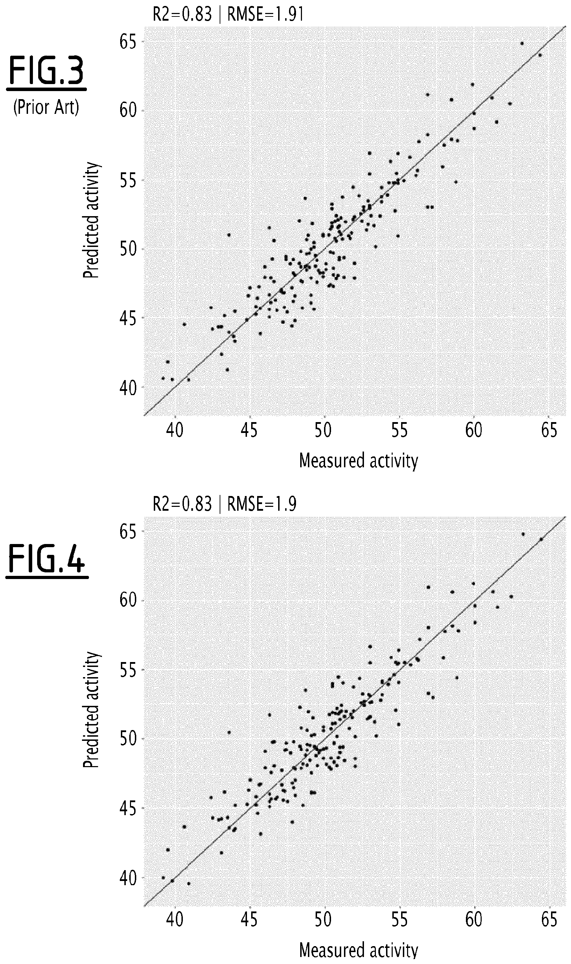

**Example 1: Cytochrome P450 (Figures 3 and 4)**

In the first example, the amino acid sequence of cytochrome P450 was encoded into a numerical sequence using a single encoding index, in particular the one identified with index number 300.

Figure 3 represents a plot of measured thermostability of cytochrome P450 variants versus thermostability predicted according to the prior art prediction method, using the encoding index identified with index number 300, while applying a Fast Fourier Transform to the encoded numerical sequence. Figure 3 therefore corresponds to FFT_Seq with index number 300.

Figure 4 represents a plot of measured thermostability of cytochrome P450 variants versus thermostability predicted with the prediction method according to the invention, using the same encoding index identified with index number 300 for two elementary numerical sequences, one elementary numerical sequence without further applying Fourier Transform to the elementary numerical sequence and the other elementary numerical sequence with further application of the Fast Fourier Transform. Figure 4 therefore corresponds to the extended numerical sequence Ext_SEQ equal to noFFT_Seq--FFT_Seq with index number 300.

Figure 3 shows the results obtained for Cytochrome P450 using the best index obtained when a Fast Fourier Transform is applied and with the prior art prediction method: cvR2 and cvRMSE are respectively 0.83 and 1.91.

With the same encoding index, Figure 4 shows better results obtained with the prediction method according to the invention: cvR2 and cvRMSE are respectively 0.83 and 1.9.

The root mean squared error RMSE, also denoted cvRMSE, and the coefficient of determination $R^2$, also denoted cvR2, are performance parameters to assess the regression model of the prediction module 26, during a validation phase with a comparison of the predicted fitness values versus corresponding measured fitness values. RMSE values varies between 0 and $+\infty$. $R^2$ value varies between 0 and 1. An accurate regression model has an RMSE close to 0 and a $R^2$ close to 1.

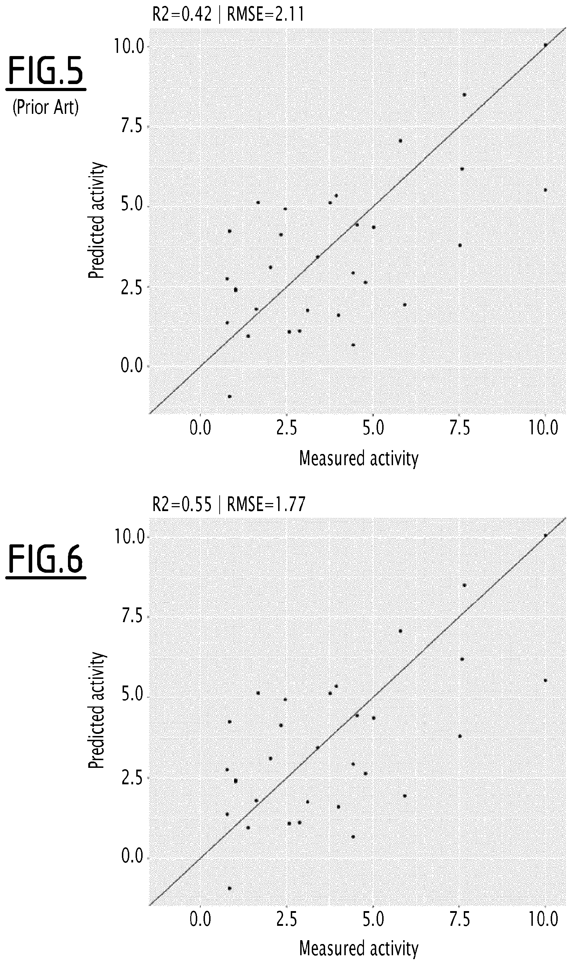

**Example 2: GLP2 mutants (Figures 5 and 6)**

In the second example, the amino acid sequence of GLP2 variants (or mutants) was encoded into a numerical sequence using a single best encoding index (index number 449) for Figure 5 and using the two best encoding indexes (index numbers 449 and 341) for Figure 6.

Figure 5 represents a plot of measured potency (fold-increase in cAMP) of GLP2 variants versus potency predicted according to the prior art prediction method, using the encoding index identified with index number 449, while applying a Fast Fourier Transform to the encoded numerical sequence. Figure 5 therefore corresponds to FFT_Seq with index number 449.

Figure 6 represents a plot of measured potency (fold-increase in cAMP) of GLP2 variants versus potency predicted with the prediction method according to the invention, using the two best encoding indexes (index numbers 449 and 341) for two elementary numerical sequences, each one with further application of the Fast Fourier Transform. Figure 6 therefore corresponds to the extended numerical sequence Ext_SEQ equal to $FFT\_Seq_{j1}$--$FFT\_Seq_{j2}$ with j1 equal to index number 449 and j2 equal to index number 341.

Figure 5 shows the results obtained with the first best index 449 alone: cvR2 and cvRMSE are respectively 0.42 and 2.11.

With the two best encoding indexes (index numbers 449 and 341), Figure 6 shows significantly better results obtained with the prediction method according to the invention: cvR2 and cvRMSE are respectively 0.55 and 1.77.

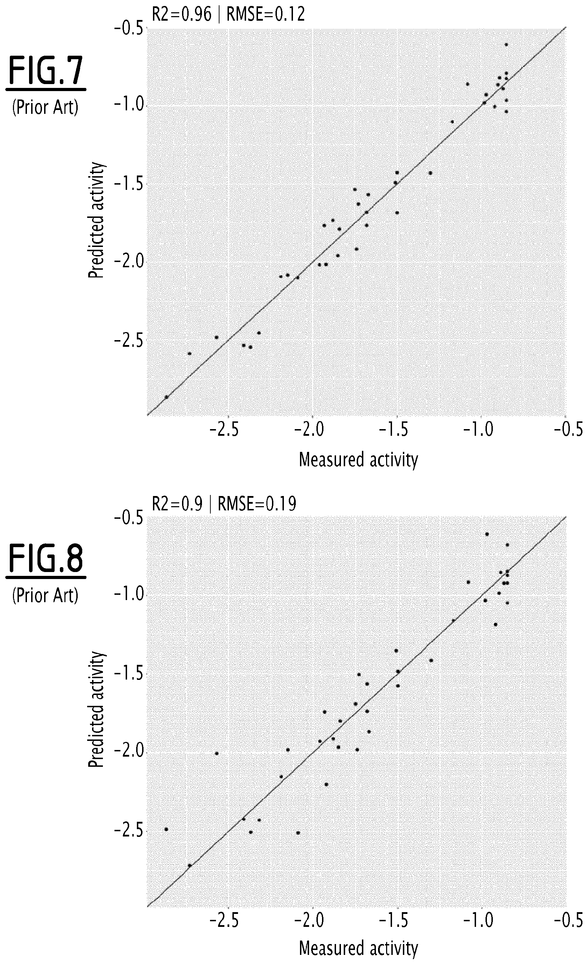

**Example 3: Epoxide hydrolase (Figures 7 to 9)**

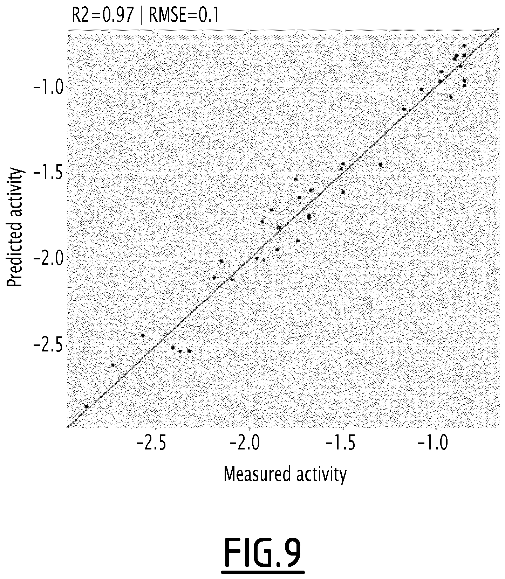

In the third example, the amino acid sequence of epoxide hydrolase variants was encoded into a numerical sequence using a single best encoding index (index number 303) for Figure 7, using a single second-best encoding index (index number 14) for Figure 8 and using the two best encoding indexes (index numbers 303 and 14) for Figure 9.

Figure 7 represents a plot of measured ΔΔG‡ of epoxide hydrolase variants versus ΔΔG‡ predicted according to the prior art prediction method, using the encoding index number 303, while applying a Fast Fourier Transform to the encoded numerical sequence. Figure 7 therefore corresponds to FFT_Seq with index number 303.

Similarly, Figure 8 represents a plot of measured ΔΔG‡ of epoxide hydrolase variants versus ΔΔG‡ predicted according to the prior art prediction method, using the encoding index number 14, while applying a Fast Fourier Transform to the encoded numerical sequence. Figure 8 therefore corresponds to FFT_Seq with index number 14.

Figure 9 represents a plot of measured ΔΔG‡ of epoxide hydrolase variants versus ΔΔG‡ predicted with the prediction method according to the invention, using the two best encoding indexes (index numbers 303 and 14) for two elementary numerical sequences, each one with further application of the Fast Fourier Transform. Figure 9 therefore corresponds to the extended numerical sequence Ext_SEQ equal to $FFT\_Seq_{j1}$--$FFT\_Seq_{j2}$ with j1 equal to index number 303 and j2 equal to index number 14.

Figure 7 shows the results obtained with the first best index 303 alone: cvR2 and cvRMSE are respectively 0.96 and 0.12. Figure 8 shows the results obtained with the second-best index 14 alone: cvR2 and cvRMSE are respectively 0.9 and 0.19.

With the two best encoding indexes (index numbers 303 and 14), Figure 9 shows slightly better results obtained with the prediction method according to the invention: cvR2 and cvRMSE are respectively 0.97 and 0.1. Thus, using two encoding indexes for obtaining the extended numerical sequence Ext_SEQ improves the quality of the prediction, once again. Even such a slight aforementioned improvement could be important when epistatic phenomenon take place.

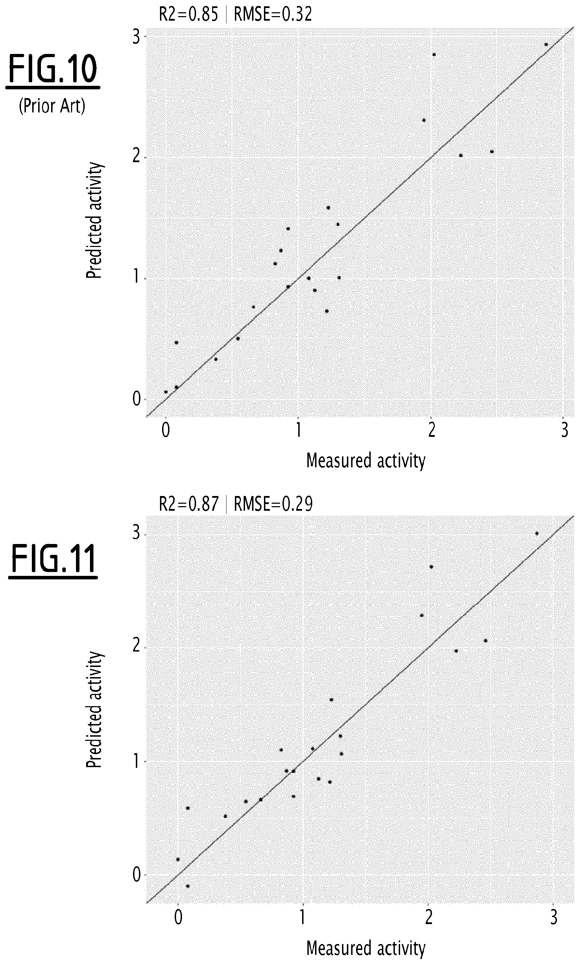

**Example 4: TNF (Figures 10 and 11)**

In the fourth example, the amino acid sequence of TNF variants was encoded into a numerical sequence using a single best encoding index (index number 203) for Figure 10 and using the two best encoding indexes (index numbers 203 and 504) for Figure 11.

Figure 10 represents a plot of measured affinity of TNF variants versus affinity predicted according to the prior art prediction method, using the encoding index with number 203, while applying a Fast Fourier Transform to the encoded numerical sequence. Figure 10 therefore corresponds to FFT_Seq with index number 203.

Figure 11 represents a plot of measured affinity of TNF variants versus affinity predicted with the prediction method according to the invention, using the two best encoding indexes (index numbers 203 and 504) for two elementary numerical sequences, each one with further application of the Fast Fourier Transform. Figure 11 therefore corresponds to the extended numerical sequence Ext_SEQ equal to $FFT\_Seq_{j1}$--$FFT\_Seq_{j2}$ with j1 equal to index number 203 and j2 equal to index number 504.

Figure 10 shows the results obtained with the first best index 203 alone: cvR2 and cvRMSE are respectively 0.85 and 0.32.

With the two best encoding indexes (index numbers 203 and 504), Figure 11 shows better results obtained with the prediction method according to the invention: cvR2 and cvRMSE are respectively 0.87 and 0.29.

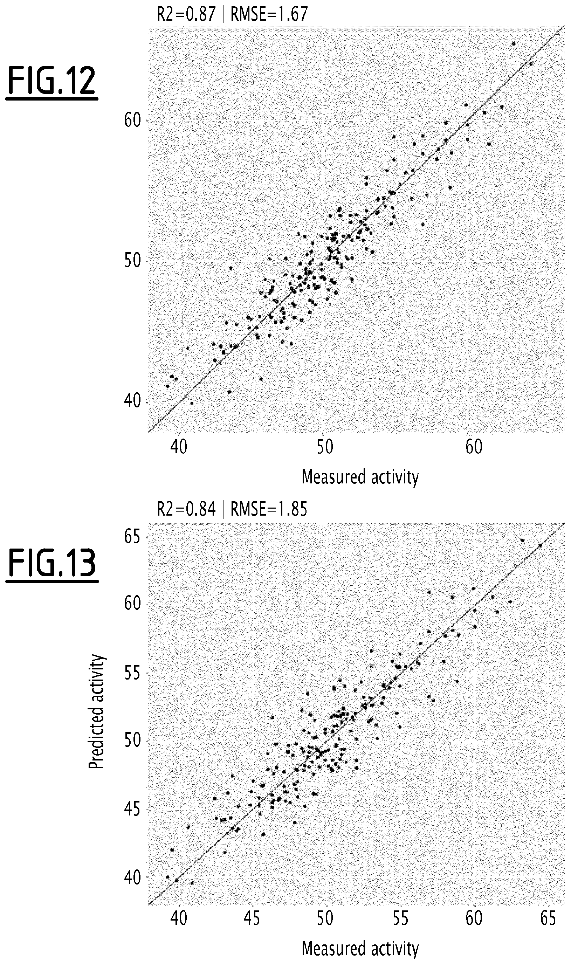

**Example 5: Cytochrome P450 (Figures 12 and 13)**

In the fifth example, the amino acid sequence of cytochrome P450 was encoded into a numerical sequence using two encoding indexes, including the best encoding index 300, for Figures 12 and 13.

Figure 12 represents a plot of measured thermostability of cytochrome P450 variants versus thermostability predicted with the prediction method according to the invention, using the two encoding indexes (index numbers 300 and 39) for two elementary numerical sequences, each one with further application of the Fast Fourier Transform. Figure 12 therefore corresponds to the extended numerical sequence Ext_SEQ equal to FFT $Seq_{j1}$-FFT_$Seq_{j2}$ with j1 equal to index number 300 and j2 equal to index number 39.

Figure 13 represents a plot of measured thermostability of cytochrome P450 variants versus thermostability predicted with the prediction method according to the invention, using the two encoding indexes (index numbers 300 and 343) for two elementary numerical sequences, one (index number 300) with further application of the Fast Fourier Transform and the other one (index number 343) without further application of the Fast Fourier Transform. Figure 13 therefore corresponds to the extended numerical sequence Ext_SEQ equal to noFFT_$Seq_{j1}$--FFT_$Seq_{j2}$ with j1 equal to index number 343 and j2 equal to index number 300.

With two encoding indexes (index numbers 300 and 39), Figure 12 shows significantly better results obtained with the prediction method according to the invention: cvR2 and cvRMSE are respectively 0.87 and 1.67 (in comparison to Figure 3 for FFT_Seq with best index number 300 where cvR2 and cvRMSE are respectively 0.83 and 1.91).

With two encoding indexes (index numbers 300 and 343), Figure 13 also shows better results obtained with the prediction method according to the invention: cvR2 and cvRMSE are respectively 0.84 and 1.85 (in comparison to Figure 3 for FFT_Seq with best index number 300 where cvR2 and cvRMSE are respectively 0.83 and 1.91).

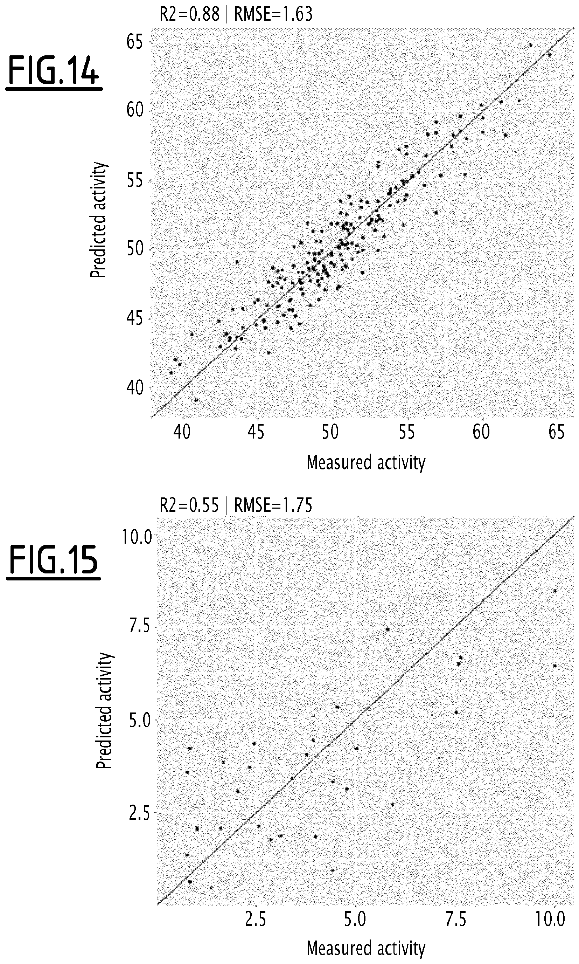

**Example 6: Cytochrome P450 (Figure 14)**

In the sixth example, the amino acid sequence of cytochrome P450 was encoded into a numerical sequence using the three best encoding indexes (index numbers 300, 39 and 226) for Figure 14.

Figure 14 represents a plot of measured thermostability of cytochrome P450 variants versus thermostability predicted with the prediction method according to the invention, using the three best encoding indexes (index numbers 300, 39 and 226) for three elementary numerical sequences, each one with further application of the Fast Fourier Transform. Figure 14 therefore corresponds to the extended numerical sequence Ext_SEQ equal to FFT_$Seq_{j1}$--FFT_$Seq_{j2}$--FFT_$Seq_{j3}$ with j1 equal to index number 300, j2 equal to index number 39 and j3 equal to index number 226.

With three encoding indexes, Figure 14 shows significantly better results obtained with the prediction method according to the invention: cvR2 and cvRMSE are respectively 0.88 and 1.63 (in comparison to Figure 3 for FFT_Seq with best index number 300 where cvR2 and cvRMSE are respectively 0.83 and 1.91).

**Example 7: Combinatorial of multiple encoding indexes**

Here, the protein sequence is encoded according to n indexes j1 to jn, the n elementary numerical sequences being each one obtained according a respective encoding index. Then a combinatorial is performed in order to find out what is the best combination of m indexes, with m varying from 2 to n.

Each combination is evaluated according to cvRMSE. The best combination corresponds to the lowest cvRMSE. In this case, the best index for one index is not necessarily the best to use for a combination of n indexes.

As an example, with GLP2 variants, the top 10 indexes (after a ranking of the 566 indexes from AAIndex) are kept. A combinatorial of 3 indexes at most is run on the top 10 indexes from the previous ranking. Since FFT_$Seq_{j1}$--FFT_$Seq_{j2}$ is equivalent to FFT_$Seq_{j2}$--FFT_$Seq_{j1}$, 175 combined extended sequences are thus obtained.

Table 7

| Indexes | cvRMSE | cvR2 |
|---|---|---|
| 440; 350; 44 | 1.99 | 0.47 |
| 440;350 | 1.99 | 0.47 |
| 440; 350; 233 | 1.99 | 0.47 |

(continued)

| Indexes | cvRMSE | cvR2 |
|---|---|---|
| 440;44 | 2.06 | 0.37 |
| 44;233 | 2.09 | 0.37 |
| 449;350 | 2.10 | 0.43 |
| 449; 350; 233 | 2.10 | 0.43 |
| 449; 350; 44 | 2.10 | 0.43 |
| 449 | 2.11 | 0.42 |
| 449;233 | 2.11 | 0.42 |

The results obtained with the prior art prediction method with the first best index 449 alone, cvR2 and cvRMSE are respectively 0.42 and 2.11.

Table 7 shows that the best obtained cvR2 and cvRMSE with the prediction method according to the invention with three indexes are respectively 0.47 and 1.99. When ten index are used in order to get $FFT\_Seq_{j1}$--$FFT\_Seq_{j2}$--...--$FFT\_Seq_{j10}$, cvRMSE jumps to 2.48 (cvR2 = 0.11).

Thus, the combinatorial of multiple indexes significantly improves the results. It should be noticed that the right number of indexes has to be determined: a combination of m indexes is not always better than a combination of n indexes with m>n.

Another example with epoxide hydrolase variants leads to similar results according to below Table 8.

Table 8

| Indexes | cvRMSE | cvR2 |
|---|---|---|
| 161; 178; 516 | 0.1051 | 0.9685 |
| 254; 178; 516 | 0.1051 | 0.9685 |
| 232; 161; 508 | 0.1123 | 0.9640 |
| 232; 254; 508 | 0.1123 | 0.9640 |
| 161; 508 | 0.1146 | 0.9629 |
| 254; 508 | 0.1146 | 0.9629 |
| 161; 254; 508 | 0.1150 | 0.9624 |
| 303; 508 | 0.1161 | 0.9624 |
| 303; 161; 508 | 0.1170 | 0.9615 |
| 303; 254; 508 | 0.1170 | 0.9615 |

It should be noticed that index 303 identified as the best one when ranking the 566 indexes of AAIndex is classified only in 38th ranking position 38 when a combinatorial is used, i.e. 37 combinations of indexes are better than index 303 alone (when considering only the top 10 in this example and when this best index 303 is included in the top 10).

**Example 8: GLP2 mutants (Figure 15)**

In the eighth example (Figure 15), the amino acid sequence of GLP2 variants was encoded into a numerical sequence using the three encoding indexes issued from three distinct families: index 449 issued from the 'other properties' family; index 341 issued from the 'alpha and turn propensities' family; and index 193 issued from the 'composition' family.

Figure 15 represents a plot of measured potency (fold-increase in cAMP) of GLP2 variants versus potency predicted with the prediction method according to the invention, using the three aforementioned encoding indexes (index numbers 449, 341 and 193) for three elementary numerical sequences, each one with further application of the Fast Fourier Transform. Figure 15 therefore corresponds to the extended numerical sequence Ext_SEQ equal to $FFT\_Seq_{j1}$--$FFT\_Seq_{j2}$--$FFT\_Seq_{j3}$ with j1 equal to index number 449, j2 equal to index number 341 and j3 equal to index number 193.

With three encoding indexes, Figure 15 shows significantly better results obtained with the prediction method according to the invention: cvR2 and cvRMSE are respectively 0.55 and 1.75 (in comparison to Figure 5 for FFT_Seq with best index number 449 where cvR2 and cvRMSE are respectively 0.42 and 2.11).

**Example 9: TNF (Figures 16 and 17)**

In the ninth example (Figures 16 and 17), the amino acid sequence of TNF variants was encoded into a numerical sequence using the three encoding indexes issued from three distinct clusters: index 203 issued from cluster C3; index 504 issued from cluster C8; and index 486 issued from cluster C5 of Figure 17.

For obtaining the classification into clusters, such as the one shown in Figure 17, the encoding indexes, such as the 566 indexes of AAIndex, are classified into different clusters using an approach for unsupervised clusterization such a K-means, fuzzy analysis clustering, partitioning around medoids, etc.

Each index is affected to a cluster based on the selected approach. A ranking on the indexes is run and in each cluster one or more top indexes are selected. As an example, a combinatorial as described above could be performed using the top NbC index where one index is chosen in one cluster (NbC = number of clusters). The clustering allows to regroup the indexes by their statistical features rather than by their biological and physicochemical features.

Figure 16 represents a plot of measured affinity of TNF variants versus affinity predicted with the prediction method according to the invention, using the three aforementioned encoding indexes (index numbers 203, 504 and 486) for three elementary numerical sequences, each one with further application of the Fast Fourier Transform. Figure 16 therefore corresponds to the extended numerical sequence Ext_SEQ equal to $FFT\_Seq_{j1}$--$FFT\_Seq_{j2}$--$FFT\_Seq_{j3}$ with j1 equal to index number 203, j2 equal to index number 504 and j3 equal to index number 486.

With three encoding indexes, Figure 16 shows significantly better results obtained with the prediction method according to the invention: cvR2 and cvRMSE are respectively 0.88 and 0.28 (in comparison to Figure 5 for FFT_Seq with best index number 449 where cvR2 and cvRMSE are respectively 0.42 and 2.11).

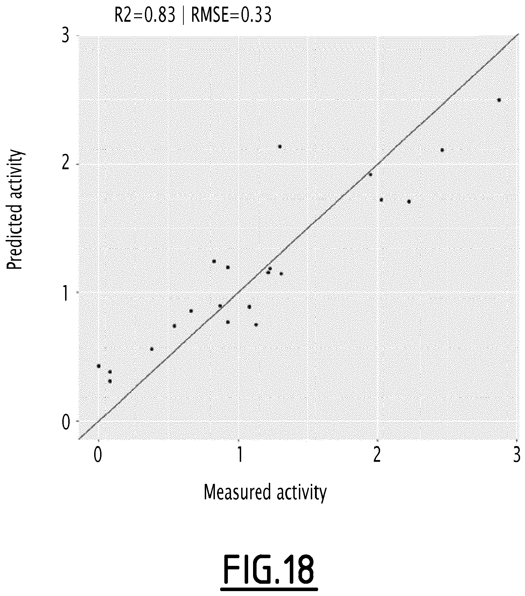

**Example 10: TNF (Figure 18)**

Alternatively, a model is built for each index and selected models (based on the cvRMSE criteria) are used to form ensemble of models in order to calculate for example a mean of the predictions of the hold out sequences with each of these models, or to use the predicted values of each model to build a new model that allow new predictions, or more generally to use different approaches of ensemble modeling such as staking, bagging, boosting.

As an example, 20 models are used based on one index at a time (i.e. 20 different indexes are used), 10 that predict efficiently an upper part of the plot and 10 that predict efficiently a down part of the plot, and a mean of the predictions is computed. The mean of the predictions is then expected to fit in a better way to the diagonal.

Table 9

| Model | Type | R2 |
|---|---|---|
| 1 | Down model | 0.47 |
| 2 | Down model | 0.45 |
| 3 | Down model | 0.63 |
| 4 | Down model | 0.46 |
| 5 | Down model | 0.45 |
| 6 | Down model | 0.51 |
| 7 | Down model | 0.53 |
| 8 | Down model | 0.49 |
| 9 | Down model | 0.41 |
| 10 | Down model | 0.50 |
| 11 | Upper model | 0.47 |
| 12 | Upper model | 0.50 |
| 13 | Upper model | 0.34 |
| 14 | Upper model | 0.56 |

(continued)

| Model | Type | R2 |
|---|---|---|
| 15 | Upper model | 0.71 |
| 16 | Upper model | 0.55 |
| 17 | Upper model | 0.61 |
| 18 | Upper model | 0.60 |
| 19 | Upper model | 0.36 |
| 20 | Upper model | 0.33 |
| Ensemble | Ensemble of upper and down models | 0.83 |

Above Table 9 provides such example results for the set of TNF variants.

Figure 18 represents a plot of measured affinity of TNF variants versus affinity predicted with the prediction method according to the invention, using the aforementioned ensemble.

With the aforementioned ensemble, Figure 18 shows significantly better results obtained with the prediction method according to the invention: cvR2 and cvRMSE are respectively 0.83 and 0.33 (in comparison to Figure 5 for FFT_Seq with best index number 449 where cvR2 and cvRMSE are respectively 0.42 and 2.11).

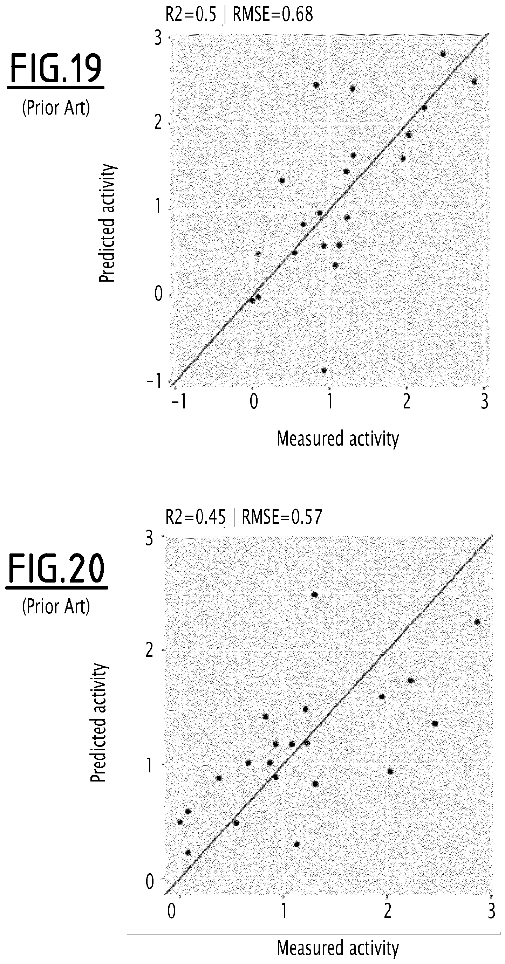

**Example 11: TNF (Figures 19 to 21)**

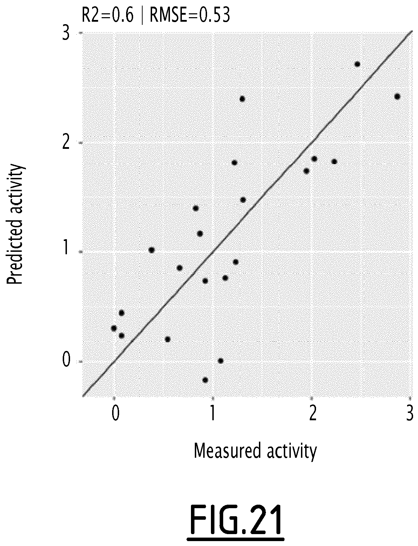

In the eleventh example, the amino acid sequence of TNF variants was encoded into a numerical sequence using a single first encoding index (index number 523) for Figure 19 corresponding to an upper model, using a single second encoding index (index number 297) for Figure 20 corresponding to a down model and using these two aforementioned encoding indexes (index numbers 523 and 297) for Figure 21.

Figure 19 represents a plot of measured affinity of TNF variants versus affinity predicted according to the prior art prediction method, using the encoding index number 523, while applying a Fast Fourier Transform to the encoded numerical sequence. Figure 19 therefore corresponds to FFT_Seq with index number 523.

Similarly, Figure 20 represents a plot of measured affinity of TNF variants versus affinity predicted according to the prior art prediction method, using the encoding index number 297, while applying a Fast Fourier Transform to the encoded numerical sequence. Figure 20 therefore corresponds to FFT_Seq with index number 297.

Figure 21 represents a plot of measured affinity of TNF variants versus affinity predicted with the prediction method according to the invention, using the two aforementioned encoding indexes (index numbers 523 and 297) for two elementary numerical sequences, each one with further application of the Fast Fourier Transform. Figure 21 therefore corresponds to the extended numerical sequence Ext_SEQ equal to $FFT\_Seq_{j1}$--$FFT\_Seq_{j2}$ with j1 equal to index number 523 and j2 equal to index number 297.

Figure 19 shows the results obtained with the first index 523 alone: cvR2 and cvRMSE are respectively 0.5 and 0.68. Figure 20 shows the results obtained with the second index 297 alone: cvR2 and cvRMSE are respectively 0.45 and 0.57.

With these two encoding indexes (index numbers 523 and 297), Figure 21 shows better results obtained with the prediction method according to the invention: cvR2 and cvRMSE are respectively 0.6 and 0.53.

**Example 12: Cytochrome P450 (Figures 22 to 24)**

In optional addition, as previously described, the prediction method according to the invention is applicable on the entire protein sequence as exemplified in the previous examples or on a selection of position(s) in the protein sequence without FFT and/or on a selection of frequencies in the protein spectrum of the FFT.

The selection of position(s) is done in a similar manner than the selection of frequency(ies) or harmonic(s), i.e. by using a filter method or a wrapper method, as previously described.

The twelfth example is an example of this optional feature, wherein the prediction method according to the invention is carried out for a selection of frequencies in the protein spectrum of the FFT, i.e. for a given set of frequency(ies) or harmonic(s), such as one or several selected harmonics corresponding to one or several frequency ranges.

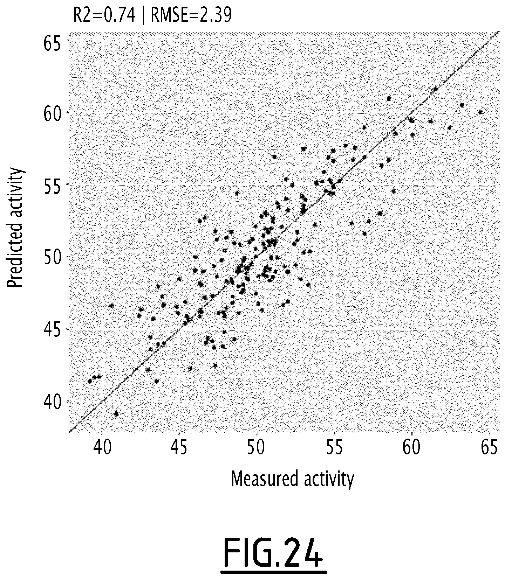

In the twelfth example, the amino acid sequence of cytochrome P450 was encoded into a numerical sequence using a single best encoding index (index number 300) for Figures 22 and 23 and using the two best encoding indexes (index numbers 300 and 343) for Figure 24.

Figure 22 represents a plot of measured thermostability of cytochrome P450 variants versus thermostability predicted according to the prior art prediction method, using the encoding index with number 300, while applying a Fast Fourier Transform to the encoded numerical sequence, but only for a given set of harmonic(s) representing a part of the whole spectrum. In this example, the set of harmonic(s) represents approximately 20% of the whole considered spectrum. The harmonics are for example numbered from 0 to 256 and the selected harmonics in this example are the following ones: 3; 7; 18; 22; 29; 33; 42; 46; 48; 58; 59; 65; 69; 79; 81; 88; 94; 99; 103; 109; 111; 112; 115; 128; 132; 134; 138; 139; 142;146; 159; 160; 163; 165; 171; 177; 182; 183; 184; 206; 214; 220; 222; 223; 224; 225; 226; 230; 235; 238; 240; 249. Figure 10 therefore corresponds to $FFT_{20\%}$_ Seq with index number 300 where $FFT_{20\%}$ denotes that the Fast Fourier Transform is applied only for a given set of frequency(ies) or harmonic(s) representing 20% of the whole spectrum.

Figure 23 represents a plot of measured thermostability of cytochrome P450 variants versus thermostability predicted with the prediction method according to the invention, using the same encoding index identified with index number 300 for two elementary numerical sequences, one elementary numerical sequence without further applying Fourier Transform to the elementary numerical sequence and the other elementary numerical sequence with further application of the Fast Fourier Transform, but only for said given set of frequency(ies) or harmonic(s) representing 20% of the whole spectrum. Figure 23 therefore corresponds to the extended numerical sequence Ext_SEQ equal to noFFT_Seq--$FFT_{20\%}$_ Seq with index number 300.

Figure 24 represents a plot of measured thermostability of cytochrome P450 variants versus thermostability predicted with the prediction method according to the invention, using the two best encoding indexes (index numbers 300 and 343) for two elementary numerical sequences, one (index number 343) without further applying Fourier Transform to the elementary numerical sequence and the other (index number 300) with further application of the Fast Fourier Transform, but only for said given set of frequency(ies) or harmonic(s) representing 20% of the whole spectrum. Figure 24 therefore corresponds to the extended numerical sequence Ext_SEQ equal to $noFFT\_Seq_{j1}$--$FFT_{20\%}\_Seq_{j2}$ with j1 equal to index number 343 and j2 equal to index number 300.

Figure 22 shows the results obtained with the best index 300 alone and $FFT_{20\%}$: cvR2 and cvRMSE are respectively 0.66 and 2.68.

With the same encoding index 300, without FFT and with $FFT_{20\%}$:, Figure 23 shows better results obtained with the prediction method according to the invention: cvR2 and cvRMSE are respectively 0.74 and 2.38.

With the two best encoding indexes (index numbers 300 and 343) and $FFT_{20\%}$ for index number 300, Figure 24 shows better results obtained with the prediction method according to the invention: cvR2 and cvRMSE are respectively 0.74 and 2.39.

Thus, R2 and RMSE between the predicted values and the measured values of several fitness, as illustrated in the aforementioned examples, show that the prediction system 10 and method according to the invention allow a more efficient prediction of different fitness values of different proteins or protein variants than the prior art prediction system and method.

**Claims**

1. Method for predicting at least one fitness value of a protein, the method being implemented on a computer and **characterized by** including the following steps:

   - calculating (100) Q elementary numerical sequences, Q being an integer greater than or equal to 2, each elementary numerical sequence depending on a respective encoding of the amino acid sequence of the protein according to a protein database (21),
   - determining (110) an extended numerical sequence, Ext_SEQ, by concatenating the Q elementary numerical sequences,

   for each fitness:

   - comparing (130) the determined extended numerical sequence, Ext_SEQ, with reference extended numerical sequences of a predetermined database (25), said database (25) containing reference extended numerical sequences for different values of said fitness,
   - predicting (130) a value of said fitness according to the comparison step.

2. The method according to claim 1, wherein at least one elementary numerical sequence is an elementary protein spectrum, the elementary protein spectrum being obtained by applying a Fourier Transform to an intermediate numerical sequence, the intermediate numerical sequence being obtained by a respective encoding of the amino acid sequence of the protein,
   the Fourier Transform being preferably a Fast Fourier Transform,
   at least one elementary protein spectrum being preferably calculated for said amino acid sequence according to a given set of frequency or frequencies.

3. The method according to claim 2, wherein each elementary protein spectrum depends on the following equation:

$$f_j = \sum_{k=0}^{N-1} x_k \cdot exp\left(\frac{-2i\pi}{N} \cdot j \cdot k\right)$$

   where j is an index-number of the elementary protein spectrum $f_j$;
   the intermediate numerical sequence includes N value(s) denoted $x_k$, with $0 \leq k \leq N-1$ and $N \geq 1$; and
   i defining the imaginary number such that $i^2 = -1$.

4. The method according to any one of the preceding claims, wherein the protein database (21) includes at least one index of numerical values, each numerical value being given for a respective amino acid; and
   wherein each encoding of the amino acid sequence of the protein is performed for a respective index, the value in the numerical sequence for each amino acid being equal to the numerical value for said amino acid in the respective index.

5. The method according to any one of the preceding claims, wherein all the elementary numerical sequences are distinct from each other.

6. The method according to claims 2, 4 and 5, wherein, among a pair of elementary numerical sequences, one differs from the other further to the applying of the Fourier Transform for only one elementary numerical sequence of the pair and/or further to a different index from the one to the other elementary numerical sequence of the pair.

7. The method according to claim 4 or 6, wherein the protein database (21) includes several indexes of numerical values, and
   wherein the method further includes a step of:

   - selecting (100) the best index(es) based on a comparison of measured fitness values for sample proteins with predicted fitness values previously obtained for said sample proteins according to each index;

   at least one encoding of the amino acid sequence of the protein being then performed using a respective selected

40

index.

8. The method according to claim 7, wherein, during the selection step, the selected index(es) are the index(es) with the smallest root mean square error(s),
   wherein the root mean square error for each index verifies the following equation:

$$RMSE_{Index\_j} = \sqrt{\sum_{i=1}^{S} \frac{(y_i - \hat{y}_{i,j})^2}{S}}$$

where $y_i$ is the measured fitness of the $i^{th}$ sample protein,
$\hat{y}_{i,j}$ is the predicted fitness of the $i^{th}$ sample protein with the $j^{th}$ index, and
S the number of sample proteins.

9. The method according to claim 7, wherein, during the selection step, the selected index(es) are the index(es) with the coefficient(s) of determination nearest to 1,
   wherein the coefficient of determination for each index verifies the following equation:

$$R^2{}_{Index\_j} = \frac{(\sum_{i=1}^{S} (y_i - \overline{y})(\hat{y}_{i,j} - \overline{\hat{y}}))^2}{\sum_{i=1}^{S} (y_i - \overline{y})^2 \sum_{i=1}^{S} (\hat{y}_{i,j} - \overline{\hat{y}})^2}$$

where $y_i$ is the measured fitness of the $i^{th}$ sample protein,
$\hat{y}_{i,j}$ is the predicted fitness of the $i^{th}$ sample protein with the $j^{th}$ index,
S the number of sample proteins,
$\overline{y}$ is an average of the measured fitness for the S sample proteins, and

$\overline{\hat{y}}$ is an average of the predicted fitness for the S sample proteins.

10. The method according to any one of the preceding claims, wherein, during the determining step (110), the elementary numerical sequences are concatenated according to a concatenation pattern for determining the extended numerical sequence, Ext_SEQ, the reference extended numerical sequences having being obtained with the same concatenation pattern.

11. The method according to claims 2, 4 and 10, wherein the concatenation pattern defines, for each elementary numerical sequence from the succession of the elementary numerical sequences to be concatenated, the respective index and the applying or not of the Fourier Transform.

12. The method according to claim 10 or 11, wherein the protein database (21) includes several indexes classified into distinct categories, and
    wherein the concatenation pattern includes indexes from at least two categories;
    each category being preferably a family associated to a protein feature, such as a protein feature chosen from among the group consisting of: alpha & turn propensities, beta propensity, composition, hydrophobicity, physico-chemical property and other protein property; or
    each category being preferably a cluster of index(es), the clusters being obtained according to statistical feature(s) of the indexes.

13. The method according to any one of the preceding claims, wherein the comparison step (130) comprises identifying, in the predetermined database (25) of reference extended numerical sequences for different values of said fitness, the reference extended numerical sequence which is the closest according to a predetermined criterion to the determined extended numerical sequence, Ext_SEQ,
    the predicted value of said fitness being then equal to the fitness value which is associated in said database with the identified reference extended numerical sequence.

14. A computer program product including software instructions which, when implemented by a computer, implement a method according to any one of the preceding claims.

15. An electronic prediction system (10) for predicting at least one fitness value of a protein, the prediction system (10) including:

- a calculation module (20) configured for calculating Q elementary numerical sequences, Q being an integer greater than or equal to 2, each elementary numerical sequence depending on a respective encoding of the amino acid sequence of the protein according to a protein database (21),
- a determination module (22) configured for determining an extended numerical sequence, Ext_SEQ, by concatenating the Q elementary numerical sequences,
- a prediction module (26) configured for, for each fitness:

    + comparing the determined extended numerical sequence, Ext_SEQ, with reference extended numerical sequences of a predetermined database (25), said database (25) containing reference extended numerical sequences for different values of said fitness,
    + predicting a value of said fitness according to said comparison.

**Patentansprüche**

1. Verfahren zum Vorhersagen von zumindest einem Fitness-Wert eines Proteins, wobei das Verfahren auf einem Computer implementiert ist und gekennzeichnet ist, indem es die folgenden Schritte aufweist:

    - Berechnen (100) von Q elementaren numerischen Sequenzen, wobei Q eine ganze Zahl ist, welche größer oder gleich 2 ist, wobei jede elementare numerische Sequenz von einer jeweiligen Kodierung der Aminosäuresequenz des Proteins gemäß einer Protein-Datenbank (21) abhängt,
    - Ermitteln (110) einer erweiterten numerischen Sequenz, Ext_SEQ, mittels Konkatenierens der Q elementaren numerischen Sequenzen,

    für jede Fitness:

    - Vergleichen (130) der ermittelten erweiterten numerischen Sequenz, Ext_SEQ, mit erweiterten numerischen Referenz-Sequenzen einer vorbestimmten Datenbank (25), wobei die besagte Datenbank (25) erweiterte numerische Referenz-Sequenzen für unterschiedliche Werte der besagten Fitness enthält,
    - Vorhersagen (130) eines Werts der besagten Fitness gemäß dem Vergleichen-Schritt.

2. Verfahren gemäß Anspruch 1, wobei zumindest eine elementare numerische Sequenz ein elementares Proteinspektrum ist, wobei das elementare Proteinspektrum mittels Anwendens einer Fourier-Transformation auf eine numerische Zwischensequenz erlangt wird, wobei die numerische Zwischensequenz mittels einer jeweiligen Kodierung der Aminosäuresequenz des Proteins erlangt wird, wobei die Fourier-Transformation vorzugsweise eine schnelle Fourier-Transformation ist, wobei zumindest ein elementares Proteinspektrum vorzugsweise für die besagte Aminosäuresequenz gemäß einem gegebenen Satz einer Frequenz oder von Frequenzen berechnet wird.

3. Verfahren gemäß Anspruch 2, wobei jedes elementare Proteinspektrum von der folgenden Gleichung abhängt:

$$f_j = \sum_{k=0}^{N-1} x_k \cdot exp\left(\frac{-2i\pi}{N} \cdot j \cdot k\right)$$

wobei j eine Index-Zahl des elementaren Proteinspektrums $f_j$ ist,
die numerische Zwischensequenz N Wert(e) aufweist, welcher/welche als $x_k$ bezeichnet wird/werden, mit $0 \leq k \leq N-1$ und $N \geq 1$, und
i die imaginäre Zahl definiert, sodass $i^2 = -1$.

**4.** Verfahren gemäß irgendeinem der vorherigen Ansprüche, wobei die Protein-Datenbank (21) zumindest einen Index von numerischen Werten aufweist, wobei jeder numerische Wert für eine jeweilige Aminosäure gegeben ist, und wobei jede Kodierung der Aminosäuresequenz des Proteins für einen jeweiligen Index durchgeführt wird, wobei der Wert in der numerischen Sequenz für jede Aminosäure gleich ist zum numerischen Wert für die besagte Aminosäure im jeweiligen Index.

**5.** Verfahren gemäß irgendeinem der vorherigen Ansprüche, wobei alle elementaren numerischen Sequenzen verschieden voneinander sind.

**6.** Verfahren gemäß den Ansprüchen 2, 4 und 5, wobei sich von einem Paar elementarer numerischer Sequenzen eine von der anderen weiter unterscheidet durch das Anwenden der Fourier-Transformation für nur eine elementare numerische Sequenz des Paars und/oder durch einen Index, welcher von der einen zur anderen elementaren numerischen Sequenz des Paares unterschiedlich ist.

**7.** Verfahren gemäß Anspruch 4 oder 6, wobei die Protein-Datenbank (21) mehrere Indizes numerischer Werte aufweist, und
wobei das Verfahren ferner aufweist einen Schritt des:

- Auswählens (100) des/der besten Index/Indizes basierend auf einem Vergleich von gemessenen Fitness-Werten für Probe-Proteine mit vorhergesagten Fitness-Werten, welche zuvor für die besagten Probe-Proteine gemäß jedem Index erlangt wurden,

wobei dann zumindest eine Kodierung der Aminosäuresequenz des Proteins unter Verwendung eines jeweiligen ausgewählten Index durchgeführt wird.

**8.** Verfahren gemäß Anspruch 7, wobei, während des Auswählen-Schritts, der/die ausgewählte/ausgewählten Index/Indizes der/die Index/Indizes mit dem/den kleinsten Quadratischer-Mittelwert-Fehler/Quadratischer-Mittelwert-Fehlern sind,
wobei der Quadratischer-Mittelwert-Fehler für jeden Index der folgenden Gleichung genügt:

$$RMSE_{Index\_j} = \sqrt{\sum_{i=1}^{S} \frac{(y_i - \hat{y}_{i,j})^2}{S}}$$

wobei $y_i$ die gemessene Fitness des i-ten Probe-Proteins ist,
$\hat{y}_{i,j}$ die vorhergesagte Fitness des i-ten Probe-Proteins mit dem j-ten Index ist, und
S die Anzahl an Probe-Proteinen ist.

**9.** Verfahren gemäß Anspruch 7, wobei, während des Auswählen-Schrittes, der/die ausgewählte/ausgewählten Index/Indizes der/die Index/Indizes mit dem/den Ermitteln-Koeffizienten, der am nächsten zu 1 ist, sind,
wobei der Ermitteln-Koeffizienten für jeden Index der folgenden Gleichung genügt:

$$R^2_{Index\_j} = \frac{(\sum_{i=1}^{S} (y_i - \overline{y})(\hat{y}_{i,j} - \overline{\hat{y}}))^2}{\sum_{i=1}^{S} (y_i - \overline{y})^2 \sum_{i=1}^{S} (\hat{y}_{i,j} - \overline{\hat{y}})^2}$$

wobei yi die gemessene Fitness des i-ten Probe-Proteins ist,
$\hat{y}_{i,j}$ die vorhergesagte Fitness des i-ten Probe-Proteins mit dem j-ten Index ist,
S die Anzahl an Probe-Proteinen ist,
$\overline{y}$ ein Mittelwert der gemessenen Fitness für die S Probe-Proteine ist und

$\overline{\hat{y}}$ ein Mittelwert der vorhergesagten Fitness für die S Probe-Proteine ist.

**10.** Verfahren gemäß irgendeinem der vorherigen Ansprüche, wobei, während des Ermitteln-Schritts (110), die elementaren numerischen Sequenzen gemäß einem Konkatenieren-Muster konkateniert werden zum Ermitteln der erweiterten numerischen Sequenz, Ext_SEQ, wobei die erweiterten numerischen Referenz-Sequenzen mit demselben Konkatenieren-Muster erlangt worden sind.

**11.** Verfahren gemäß den Ansprüchen 2, 4 und 10, wobei das Konkatenieren-Muster, für jede elementare numerische Sequenz von der Abfolge der zu konkatenierenden elementaren numerischen Sequenzen, den jeweiligen Index und das Anwenden oder Nicht-Anwenden der Fourier-Transformation definiert.

**12.** Verfahren gemäß Anspruch 10 oder 11, wobei die Protein-Datenbank (21) mehrere Indizes aufweist, welche in unterschiedliche Kategorien klassifiziert sind, und
wobei das Konkatenieren-Muster Indizes aus zumindest zwei Kategorien aufweist,
wobei jede Kategorie vorzugsweise eine Familie ist, welche mit einem Protein-Merkmal assoziiert ist, wie zum Beispiel einem Protein-Merkmal, welches aus der Gruppe ausgewählt ist, welche besteht aus: Alpha- & Windungs-Neigung, Beta-Neigung, Zusammensetzung, Hydrophobizität, physikochemische Eigenschaft und andere Protein-Eigenschaft, oder
jede Kategorie vorzugsweise eine Gruppe eines Index/von Indizes ist, wobei die Gruppen gemäß einem statistischen Merkmal/statistischen Merkmalen der Indizes erlangt werden.

**13.** Verfahren gemäß irgendeinem der vorherigen Ansprüche, wobei der Vergleichen-Schritt (130) aufweist Identifizieren, in der vorbestimmten Datenbank (25) von erweiterten numerischen Referenz-Sequenzen für unterschiedliche Werte der besagten Fitness, der erweiterten numerischen Referenz-Sequenz, welche gemäß einem vorbestimmten Kriterium die nächste zur ermittelten erweiterten numerischen Sequenz, Ext_SEQ, ist,
wobei der vorhergesagte Wert der besagten Fitness dann gleich dem Fitness-Wert ist, welcher in der besagten Datenbank mit der identifizierten erweiterten numerischen Referenz-Sequenz assoziiert ist.

**14.** Computerprogrammprodukt, welches Softwareanweisungen aufweist, welche, wenn sie mittels eines Computers implementiert werden, ein Verfahren gemäß irgendeinem der vorherigen Ansprüche implementieren.

**15.** Elektronisches Vorhersagesystem (10) zum Vorhersagen von zumindest einem Fitness-Wert eines Proteins, wobei das Vorhersagesystem (10) aufweist:

- ein Berechnen-Modul (20), welches eingerichtet ist zum Berechnen von Q elementaren numerischen Sequenzen, wobei Q eine ganze Zahl ist, welche größer oder gleich 2 ist, wobei jede elementare numerische Sequenz von einer jeweiligen Kodierung der Aminosäuresequenz des Proteins gemäß einer Protein-Datenbank (21) abhängt,
- ein Ermitteln-Modul (22), welches eingerichtet ist zum Ermitteln einer erweiterten numerischen Sequenz, Ext_SEQ, mittels Konkatenierens der Q elementaren numerischen Sequenzen,
- ein Vorhersagen-Modul (26), welches eingerichtet ist zum, für jede Fitness:

+ Vergleichen der ermittelten erweiterten numerischen Sequenz, Ext_SEQ, mit erweiterten numerischen Referenz-Sequenzen einer vorbestimmten Datenbank (25), wobei die besagte Datenbank (25) erweiterte numerische Referenz-Sequenzen für unterschiedliche Werte der besagten Fitness enthält,
+ Vorhersagen eines Werts der besagten Fitness gemäß dem besagten Vergleich.

## Revendications

**1.** Procédé de prédiction d'au moins une valeur d'activité d'une protéine, le procédé étant mis en œuvre sur un ordinateur et **caractérisé en ce qu'**il comprend les étapes suivantes :

- calcul (100) de Q séquences numériques élémentaires, Q étant un entier supérieur ou égal à 2, chaque séquence numérique élémentaire dépendant d'un codage respectif de la séquence d'acides aminés de la protéine selon une base de données de protéines (21),
- détermination (110) d'une séquence numérique étendue, Ext_SEQ, par concaténation des Q séquences numériques élémentaires,

pour chaque activité :

- comparaison (130) de la séquence numérique étendue déterminée, Ext_SEQ, à des séquences numériques étendues de référence d'une base de données prédéterminée (25), ladite base de données (25) contenant des séquences numériques étendues de référence pour différentes valeurs de ladite activité,
- prédiction (130) d'une valeur de ladite activité en fonction de l'étape de comparaison.

2. Procédé selon la revendication 1, dans lequel au moins une séquence numérique élémentaire est un spectre de protéine élémentaire, le spectre de protéine élémentaire étant obtenu par application d'une transformée de Fourier à une séquence numérique intermédiaire, la séquence numérique intermédiaire étant obtenue par un codage respectif de la séquence d'acides aminés de la protéine,
la transformée de Fourier étant de préférence une transformée de Fourier rapide, au moins un spectre de protéine élémentaire étant de préférence calculé pour ladite séquence d'acides aminés en fonction d'un ensemble donné de fréquence(s).

3. Procédé selon la revendication 2, dans lequel chaque spectre de protéine élémentaire dépend de l'équation suivante :

$$f_j = \sum_{k=0}^{N-1} x_k \cdot exp\left(\frac{-2i\pi}{N} \cdot j \cdot k\right)$$

où j est un numéro d'index du spectre de protéine élémentaire $f_j$ ;
la séquence numérique intermédiaire comprend N valeur(s) désignées par $x_k$, avec $0 \leq k \leq N\text{-}1$ et $N \geq 1$ ; et
i définissant le nombre imaginaire tel que $i^2 = -1$.

4. Procédé selon l'une quelconque des revendications précédentes, dans lequel la base de données de protéines (21) comprend au moins un index de valeurs numériques, chaque valeur numérique étant donnée pour un acide aminé respectif ; et
dans lequel chaque codage de la séquence d'acides aminés de la protéine est effectué pour un index respectif, la valeur dans la séquence numérique pour chaque acide aminé étant égale à la valeur numérique pour ledit acide aminé dans l'index respectif.

5. Procédé selon l'une quelconque des revendications précédentes, dans lequel toutes les séquences numériques élémentaires sont distinctes les unes des autres.

6. Procédé selon les revendications 2, 4 et 5, dans lequel, parmi une paire de séquences numériques élémentaires, l'un diffère de l'autre suite à l'application de la transformée de Fourier pour une seule séquence numérique élémentaire de la paire et/ou suite à un index différent de l'une ou l'autre séquence numérique élémentaire de la paire.

7. Procédé selon la revendication 4 ou 6, dans lequel la base de données de protéines (21) comprend plusieurs index de valeurs numériques, et
dans lequel le procédé comprend en outre une étape de sélection (100) du ou des meilleur(s) index sur la base d'une comparaison de valeurs mesurées d'activité pour des protéines d'échantillon à des valeurs prédites d'activité précédemment obtenues pour lesdites protéines d'échantillon en fonction de chaque index ;
au moins un codage de la séquence d'acides aminés de la protéine étant ensuite effectué en utilisant un index respectif sélectionné.

8. Procédé selon la revendication 7, dans lequel, pendant l'étape de sélection, le(s) index sélectionné(s) sont le(s) index avec la ou les plus faible(s) erreur(s) quadratique(s) moyenne(s),
dans lequel l'erreur quadratique moyenne pour chaque index satisfait à l'équation suivante :

$$RMSE_{Index\_j} = \sqrt{\sum_{i=1}^{S} \frac{(y_i - \hat{y}_{i,j})^2}{S}}$$

où $y_i$ est l'activité mesurée de la $i^{\text{ième}}$ protéine d'échantillon,
$\hat{y}_{i,j}$ est l'activité prédite de la $i^{\text{ième}}$ protéine d'échantillon avec le $j^{\text{ième}}$ index, et

S est le nombre de protéines d'échantillon.

9. Procédé selon la revendication 7, dans lequel, pendant l'étape de sélection, le(s) index sélectionné(s) sont le(s) index avec le(s) coefficient(s) de détermination les plus proches de 1,
dans lequel le coefficient de détermination pour chaque index satisfait à l'équation suivante :

$$R^2{}_{Index\_j} = \frac{(\sum\limits_{i=1}^{S}(y_i - \overline{y})(\hat{y}_{i,j} - \overline{\hat{y}}))^2}{\sum\limits_{i=1}^{S}(y_i - \overline{y})^2 \sum\limits_{i=1}^{S}(\hat{y}_{i,j} - \overline{\hat{y}})^2}$$

où $y_i$ est l'activité mesurée de la $i^{ème}$ protéine d'échantillon,
$\hat{y}_{i,j}$ est l'activité prédite de la $i^{ème}$ protéine d'échantillon avec le $j^{ème}$ index,
S est le nombre de protéines d'échantillon,
$\overline{y}$ est une moyenne de l'activité mesurée pour les S protéines d'échantillon, et

$\overline{\hat{y}}$ est une moyenne de l'activité prédite pour les S protéines d'échantillon.

10. Procédé selon l'une quelconque des revendications précédentes, dans lequel, pendant l'étape de détermination (110), les séquences numériques élémentaires sont concaténées selon un motif de concaténation pour déterminer la séquence numérique étendue, Ext_SEQ, les séquences numériques étendues de référence ayant été obtenues avec le même motif de concaténation.

11. Procédé selon revendications 2, 4 et 10, dans lequel le motif de concaténation définit, pour chaque séquence numérique élémentaire de la succession des séquences numériques élémentaires à concaténer, l'index respectif et l'application ou non de la transformée de Fourier.

12. Procédé selon la revendication 10 ou 11, dans lequel la base de données de protéines (21) comprend plusieurs index classés dans des catégories distinctes, et
dans lequel le motif de concaténation comprend des index d'au moins deux catégories ;
chaque catégorie étant de préférence une famille associée à une caractéristique de protéine, telle qu'une caractéristique de protéine choisie parmi le groupe constitué de : propensions alpha et tour, propension bêta, composition, hydrophobicité, propriété physicochimique et autre propriété de protéine ; ou
chaque catégorie étant de préférence un groupe d'index, les groupes étant obtenus en fonction de caractéristique(s) statistique(s) des index.

13. Procédé selon l'une quelconque des revendications précédentes, dans lequel l'étape de comparaison (130) comprend l'identification, dans la base de données prédéterminée (25) de séquences numériques étendues de référence pour différentes valeurs de ladite activité, la séquence numérique étendue de référence qui est la plus proche selon un critère prédéterminé de la séquence numérique étendue déterminée, Ext_SEQ,
la valeur prédite de ladite activité étant alors égale à la valeur d'activité qui est associée dans ladite base de données à la séquence numérique étendue de référence identifiée.

14. Produit de programme informatique comprenant des instructions de logiciel qui, lorsqu'elles sont mises en œuvre par un ordinateur, mettent en œuvre un procédé selon l'une quelconque des revendications précédentes.

15. Système de prédiction électronique (10) pour prédire au moins une valeur d'activité d'une protéine, le système de prédiction (10) comprenant :

- un module de calcul (20) configuré pour calculer Q séquences numériques élémentaires, Q étant un entier supérieur ou égal à 2, chaque séquence numérique élémentaire dépendant d'un codage respectif de la séquence d'acides aminés de la protéine selon une base de données de protéines (21),
- un module de détermination (22) configuré pour déterminer une séquence numérique étendue, Ext_SEQ, par concaténation des Q séquences numériques élémentaires,
- un module de prédiction (26) configuré pour, pour chaque activité :

+ comparer la séquence numérique étendue déterminée, Ext_SEQ, à des séquences numériques étendues de référence d'une base de données prédéterminée (25), ladite base de données (25) contenant des séquences numériques étendues de référence pour différentes valeurs de ladite activité,

+ prédire une valeur de ladite activité en fonction de ladite comparaison.

## FIG.1

## FIG.2

FIG.3
(Prior Art)

R2=0.83 | RMSE=1.91

FIG.4

R2=0.83 | RMSE=1.9

R2=0.42 | RMSE=2.11

**FIG.5**
(Prior Art)

R2=0.55 | RMSE=1.77

**FIG.6**

FIG.7
(Prior Art)

R2=0.96 | RMSE=0.12

Measured activity

FIG.8
(Prior Art)

R2=0.9 | RMSE=0.19

Measured activity

FIG.9

R2=0.85 | RMSE=0.32

**FIG.10**
(Prior Art)

R2=0.87 | RMSE=0.29

**FIG.11**

R2=0.87 | RMSE=1.67

FIG.12

Measured activity

R2=0.84 | RMSE=1.85

FIG.13

Measured activity

FIG.14

R2=0.88 | RMSE=1.63

FIG.15

R2=0.55 | RMSE=1.75

R2=0.88 | RMSE=0.28

## FIG.16

## FIG.17

FIG.18

R2=0.5 | RMSE=0.68

## FIG.19
### (Prior Art)

R2=0.45 | RMSE=0.57

## FIG.20
### (Prior Art)

**FIG.21**

R2=0.66 | RMSE=2.68

FIG.22
(Prior Art)

R2=0.74 | RMSE=2.38

FIG.23

FIG.24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016166253 A1

**Non-patent literature cited in the description**

- **DAMBORSKY J.** *Prot. Eng.,* January 1998, vol. 11 (1), 21-30
- **FOX R. et al.** *Protein Eng.,* 2003, vol. 16 (8), 589-97
- **FOX R.** *Journal of Theoretical Biology,* 2005, vol. 234, 187-199
- **MINSHULL J. et al.** *Curr Opin Chem Biol.,* April 2005, vol. 9 (2), 202-9
- **FOX R. et al.** *Nature Biotechnology,* 2007, vol. 25 (3), 338-344
- **FOX R. ; HUISMAN GW.** *Trends Biotechnol,* March 2008, vol. 26 (3), 132-8
- **COSIC I.** *IEEE Trans Biomed Eng.,* 1994, vol. 41 (12), 1101-14
- **NWANKWO N. ; SEKER H.** *J Proteomics Bioinform,* 2011, vol. 4 (12), 260-268
- **KAWASHIMA, S. ; KANEHISA, M.** *Nucleic Acids Res.,* 2000, vol. 28 (1), 374
- **KAWASHIMA, S. et al.** *Nucleic Acids Res.,* January 2008, vol. 36
- **VELJKOVIC V et al.** *IEEE Trans Biomed Eng.,* May 1985, vol. 32 (5), 337-41
- **VIARI A et al.** *Comput Appl Biosci,* April 1990, vol. 6 (2), 71-80
- **VELJKOVIC V. et al.** *BMC Struct Biol.,* 07 April 2009, vol. 9, 21
- **VELJKOVIC V. et al.** *BMC Struct Biol.,* 28 September 2009, vol. ;9, 62
- **COSIC I.** The Resonant Recognition Model of Macromolecular Bioactivity Birkhauser. Verlag, 1997
- **LIANG, G. ; LIU, Y. ; SHI, B. ; ZHAO, J. ; ZHENG, J.** n index for characterization of natural and non-natural amino acids for peptidomimetics. *PloS one,* 2013, vol. 8 (7), e67844
- **T. M. PHUONG ; Z. LIN ; R. B. ALTMAN.** Choosing SNPs using feature selection. *IEEE Computational Systems Bioinformatics Conference,* 2005, 301-309
- **LI, Y. ; DRUMMOND, D. A. ; SAWAYAMA, A. M. ; SNOW, C. D. ; BLOOM, J. D. ; ARNOLD, F. H.** A diverse family of thermostable cytochrome P450s created by recombination of stabilizing fragments. *Nature biotechnology,* 2007, vol. 25 (9), 1051
- **REETZ, M. T. ; SANCHIS, J.** Constructing and analyzing the fitness landscape of an experimental evolutionary process. *ChemBioChem,* 2008, vol. 9 (14), 2260-2267
- **LAKOVOU, K. ; KAZANIS, M. ; VAVAYANNIS, A. ; RUNI, G. ; ROMEO, M. R. ; MASSARELLI, P. ; MORI, T.** Synthesis of oxypropanolamine derivatives of 3, 4-dihydro-2H-1, 4-benzoxazine, β-adrenergic affinity, inotropic, chronotropic and coronary vasodilating activities. *European journal of medicinal chemistry,* 1999, vol. 34 (11), 903-917
- **MUKAI Y et al.** *J Mol Biol.,* 30 January 2009, vol. 385 (4), 1221-9

SEQUENCE LISTING

<110> PEACCEL

<120> Method and electronic system for predicting at least one fitness value of a protein via an extended numerical sequence, related computer program product

<130> BET18P2168

<160> 6

<170> PatentIn version 3.5

<210> 1
<211> 464
<212> PRT
<213> Bacillus megaterium

<400> 1

```
Met Thr Ile Lys Glu Met Pro Gln Pro Lys Thr Phe Gly Glu Leu Lys
1               5               10              15

Asn Leu Pro Leu Leu Asn Thr Asp Lys Pro Val Gln Ala Leu Met Lys
        20              25              30

Ile Ala Asp Glu Leu Gly Glu Ile Phe Lys Phe Glu Ala Pro Gly Arg
        35              40              45

Val Thr Arg Tyr Leu Ser Ser Gln Arg Leu Ile Lys Glu Ala Cys Asp
    50              55              60

Glu Ser Arg Phe Asp Lys Asn Leu Ser Gln Ala Leu Lys Phe Val Arg
65              70              75              80

Asp Phe Ala Gly Asp Gly Leu Ala Thr Ser Trp Thr His Glu Lys Asn
            85              90              95

Trp Lys Lys Ala His Asn Ile Leu Leu Pro Ser Phe Ser Gln Gln Ala
        100             105             110

Met Lys Gly Tyr His Ala Met Met Val Asp Ile Ala Val Gln Leu Val
        115             120             125

Gln Lys Trp Glu Arg Leu Asn Ala Asp Glu His Ile Glu Val Pro Glu
    130             135             140

Asp Met Thr Arg Leu Thr Leu Asp Thr Ile Gly Leu Cys Gly Phe Asn
145             150             155             160

Tyr Arg Phe Asn Ser Phe Tyr Arg Asp Gln Pro His Pro Phe Ile Thr
            165             170             175
```

```
Ser Met Val Arg Ala Leu Asp Glu Ala Met Asn Lys Leu Gln Arg Ala
        180                 185                 190

Asn Pro Asp Asp Pro Ala Tyr Asp Glu Asn Lys Arg Gln Phe Gln Glu
        195                 200                 205

Asp Ile Lys Val Met Asn Asp Leu Val Asp Lys Ile Ile Ala Asp Arg
        210                 215                 220

Lys Ala Ser Gly Glu Gln Ser Asp Asp Leu Leu Thr His Met Leu Asn
225                 230                 235                 240

Gly Lys Asp Pro Glu Thr Gly Glu Pro Leu Asp Asp Glu Asn Ile Arg
                245                 250                 255

Tyr Gln Ile Ile Thr Phe Leu Ile Ala Gly His Glu Thr Thr Ser Gly
                260                 265                 270

Leu Leu Ser Phe Ala Leu Tyr Phe Leu Val Lys Asn Pro His Val Leu
                275                 280                 285

Gln Lys Ala Ala Glu Glu Ala Ala Arg Val Leu Val Asp Pro Val Pro
        290                 295                 300

Ser Tyr Lys Gln Val Lys Gln Leu Lys Tyr Val Gly Met Val Leu Asn
305                 310                 315                 320

Glu Ala Leu Arg Leu Trp Pro Thr Ala Pro Ala Phe Ser Leu Tyr Ala
                325                 330                 335

Lys Glu Asp Thr Val Leu Gly Gly Glu Tyr Pro Leu Glu Lys Gly Asp
                340                 345                 350

Glu Leu Met Val Leu Ile Pro Gln Leu His Arg Asp Lys Thr Ile Trp
                355                 360                 365

Gly Asp Asp Val Glu Glu Phe Arg Pro Glu Arg Phe Glu Asn Pro Ser
        370                 375                 380

Ala Ile Pro Gln His Ala Phe Lys Pro Phe Gly Asn Gly Gln Arg Ala
385                 390                 395                 400

Cys Ile Gly Gln Gln Phe Ala Leu His Glu Ala Thr Leu Val Leu Gly
                405                 410                 415

Met Met Leu Lys His Phe Asp Phe Glu Asp His Thr Asn Tyr Glu Leu
```

31

```
              420                    425                        430


        Asp Ile Lys Glu Thr Leu Thr Leu Lys Pro Glu Gly Phe Val Val Lys
                435                    440                    445


        Ala Lys Ser Lys Lys Ile Pro Leu Gly Gly Ile Pro Ser Pro Ser Thr
                450                    455                    460
```

<210> 2
<211> 466
<212> PRT
<213> Bacillus megaterium

<400> 2

```
Lys Glu Thr Ser Pro Ile Pro Gln Pro Lys Thr Phe Gly Pro Leu Gly
1               5               10              15

Asn Leu Pro Leu Ile Asp Lys Asp Lys Pro Thr Leu Ser Leu Ile Lys
            20              25              30

Leu Ala Glu Glu Gln Gly Pro Ile Phe Gln Ile His Thr Pro Ala Gly
        35              40              45

Thr Thr Ile Val Val Ser Gly His Glu Leu Val Lys Glu Val Cys Asp
    50              55              60

Glu Glu Arg Phe Asp Lys Ser Ile Glu Gly Ala Leu Glu Lys Val Arg
65              70              75              80

Ala Phe Ser Gly Asp Gly Leu Ala Thr Ser Trp Thr His Glu Pro Asn
            85              90              95

Trp Arg Lys Ala His Asn Ile Leu Met Pro Thr Phe Ser Gln Arg Ala
        100             105             110

Met Lys Asp Tyr His Glu Lys Met Val Asp Ile Ala Val Gln Leu Ile
        115             120             125

Gln Lys Trp Ala Arg Leu Asn Pro Asn Glu Ala Val Asp Val Pro Gly
    130             135             140

Asp Met Thr Arg Leu Thr Leu Asp Thr Ile Gly Leu Cys Gly Phe Asn
145             150             155             160

Tyr Arg Phe Asn Ser Tyr Tyr Arg Glu Thr Pro His Pro Phe Ile Asn
            165             170             175

Ser Met Val Arg Ala Leu Asp Glu Ala Met His Gln Met Gln Arg Leu
```

```
                    180                      185                          190


        Asp Val Gln Asp Lys Leu Met Val Arg Thr Lys Arg Gln Phe Arg Tyr
                    195                  200              205


        Asp Ile Gln Thr Met Phe Ser Leu Val Asp Ser Ile Ile Ala Glu Arg
                    210             215              220


        Arg Ala Asn Gly Asp Gln Asp Glu Lys Asp Leu Leu Ala Arg Met Leu
        225                 230              235                      240


        Asn Val Glu Asp Pro Glu Thr Gly Glu Lys Leu Asp Asp Glu Asn Ile
                            245              250              255


        Arg Phe Gln Ile Ile Thr Phe Leu Ile Ala Gly His Glu Thr Thr Ser
                    260              265              270


        Gly Leu Leu Ser Phe Ala Thr Tyr Phe Leu Leu Lys His Pro Asp Lys
                    275              280              285


        Leu Lys Lys Ala Tyr Glu Glu Val Asp Arg Val Leu Thr Asp Ala Ala
            290                 295              300


        Pro Thr Tyr Lys Gln Val Leu Glu Leu Thr Tyr Ile Arg Met Ile Leu
        305                 310              315                      320


        Asn Glu Ser Leu Arg Leu Trp Pro Thr Ala Pro Ala Phe Ser Leu Tyr
                        325              330              335


        Pro Lys Glu Asp Thr Val Ile Gly Gly Lys Phe Pro Ile Thr Thr Asn
                    340              345              350


        Asp Arg Ile Ser Val Leu Ile Pro Gln Leu His Arg Asp Arg Asp Ala
                    355              360              365


        Trp Gly Lys Asp Ala Glu Glu Phe Arg Pro Glu Arg Phe Glu His Gln
            370             375              380


        Asp Gln Val Pro His His Ala Tyr Lys Pro Phe Gly Asn Gly Gln Arg
        385                 390              395                      400


        Ala Cys Ile Gly Met Gln Phe Ala Leu His Glu Ala Thr Leu Val Leu
                        405              410                      415


        Gly Met Ile Leu Lys Tyr Phe Thr Leu Ile Asp His Glu Asn Tyr Glu
                    420              425              430
```

EP 3 598 327 B1

Leu Asp Ile Lys Gln Thr Leu Thr Leu Lys Pro Gly Asp Phe His Ile
        435                 440             445

Ser Val Gln Ser Arg His Gln Glu Ala Ile His Ala Asp Val Gln Ala
        450                 455             460

Ala Glu
465

<210> 3
<211> 466
<212> PRT
<213> Bacillus megaterium

<400> 3

Lys Gln Ala Ser Ala Ile Pro Gln Pro Lys Thr Tyr Gly Pro Leu Lys
1               5               10              15

Asn Leu Pro His Leu Glu Lys Glu Gln Leu Ser Gln Ser Leu Trp Arg
        20              25              30

Ile Ala Asp Glu Leu Gly Pro Ile Phe Arg Phe Asp Phe Pro Gly Val
        35              40              45

Ser Ser Val Phe Val Ser Gly His Asn Leu Val Ala Glu Val Cys Asp
        50              55              60

Glu Lys Arg Phe Asp Lys Asn Leu Gly Lys Gly Leu Gln Lys Val Arg
65              70              75              80

Glu Phe Gly Gly Asp Gly Leu Ala Thr Ser Trp Thr His Glu Pro Asn
                85              90              95

Trp Gln Lys Ala His Arg Ile Leu Leu Pro Ser Phe Ser Gln Lys Ala
        100             105             110

Met Lys Gly Tyr His Ser Met Met Leu Asp Ile Ala Thr Gln Leu Ile
        115             120             125

Gln Lys Trp Ser Arg Leu Asn Pro Asn Glu Glu Ile Asp Val Ala Asp
        130             135             140

Asp Met Thr Arg Leu Thr Leu Asp Thr Ile Gly Leu Cys Gly Phe Asn
145             150             155             160

Tyr Arg Phe Asn Ser Phe Tyr Arg Asp Ser Gln His Pro Phe Ile Thr
                165             170             175

35

Ser Met Leu Arg Ala Leu Lys Glu Ala Met Asn Gln Ser Lys Arg Leu
        180                 185                 190

Gly Leu Gln Asp Lys Met Met Val Lys Thr Lys Leu Gln Phe Gln Lys
        195                 200                 205

Asp Ile Glu Val Met Asn Ser Leu Val Asp Arg Met Ile Ala Glu Arg
        210                 215                 220

Lys Ala Asn Pro Asp Glu Asn Ile Lys Asp Leu Leu Ser Leu Met Leu
225                 230                 235                 240

Tyr Ala Lys Asp Pro Val Thr Gly Glu Thr Leu Asp Asp Glu Asn Ile
                245                 250                 255

Arg Tyr Gln Ile Ile Thr Phe Leu Ile Ala Gly His Glu Thr Thr Ser
        260                 265                 270

Gly Leu Leu Ser Phe Ala Ile Tyr Cys Leu Leu Thr His Pro Glu Lys
        275                 280                 285

Leu Lys Lys Ala Gln Glu Glu Ala Asp Arg Val Leu Thr Asp Asp Thr
        290                 295                 300

Pro Glu Tyr Lys Gln Ile Gln Gln Leu Lys Tyr Ile Arg Met Val Leu
305                 310                 315                 320

Asn Glu Thr Leu Arg Leu Tyr Pro Thr Ala Pro Ala Phe Ser Leu Tyr
                325                 330                 335

Ala Lys Glu Asp Thr Val Leu Gly Gly Glu Tyr Pro Ile Ser Lys Gly
        340                 345                 350

Gln Pro Val Thr Val Leu Ile Pro Lys Leu His Arg Asp Gln Asn Ala
        355                 360                 365

Trp Gly Pro Asp Ala Glu Asp Phe Arg Pro Glu Arg Phe Glu Asp Pro
        370                 375                 380

Ser Ser Ile Pro His His Ala Tyr Lys Pro Phe Gly Asn Gly Gln Arg
385                 390                 395                 400

Ala Cys Ile Gly Met Gln Phe Ala Leu Gln Glu Ala Thr Met Val Leu
                405                 410                 415

Gly Leu Val Leu Lys His Phe Glu Leu Ile Asn His Thr Gly Tyr Glu
        420                 425                 430

36

```
Leu Lys Ile Lys Glu Ala Leu Thr Ile Lys Pro Asp Asp Phe Lys Ile
        435             440             445
```

```
Thr Val Lys Pro Arg Lys Thr Ala Ala Ile Asn Val Gln Arg Lys Glu
        450             455             460
```

```
Gln Ala
465
```

<210> 4
<211> 33
<212> PRT
<213> Homo sapiens

<400> 4

```
His Ala Asp Gly Ser Phe Ser Asp Glu Met Asn Thr Ile Leu Asp Asn
1               5               10              15
```

```
Leu Ala Ala Arg Asp Phe Ile Asn Trp Leu Ile Gln Thr Lys Ile Thr
        20              25              30
```

```
Asp
```

<210> 5
<211> 398
<212> PRT
<213> Aspergillus niger

<400> 5

```
Asn His Lys Ala Phe Ala Lys Phe Pro Ser Ser Ala Ser Ile Ser Pro
1               5               10              15
```

```
Asn Pro Phe Thr Val Ser Ile Pro Asp Glu Gln Leu Asp Asp Leu Lys
        20              25              30
```

```
Thr Leu Val Arg Leu Ser Lys Ile Ala Pro Pro Thr Tyr Glu Ser Leu
        35              40              45
```

```
Gln Ala Asp Gly Arg Phe Gly Ile Thr Ser Glu Trp Leu Thr Thr Met
        50              55              60
```

```
Arg Glu Lys Trp Leu Ser Glu Phe Asp Trp Arg Pro Phe Glu Ala Arg
65              70              75              80
```

```
Leu Asn Ser Phe Pro Gln Phe Thr Thr Glu Ile Glu Gly Leu Thr Ile
                85              90              95
```

```
His Phe Ala Ala Leu Phe Ser Glu Arg Glu Asp Ala Val Pro Ile Ala
        100                 105                 110

Leu Leu His Gly Trp Pro Gly Ser Phe Val Glu Phe Tyr Pro Ile Leu
        115                 120                 125

Gln Leu Phe Arg Glu Glu Tyr Thr Pro Glu Thr Leu Pro Phe His Leu
        130                 135                 140

Val Val Pro Ser Leu Pro Gly Tyr Thr Phe Ser Ser Gly Pro Pro Leu
145                 150                 155                 160

Asp Lys Asp Phe Gly Leu Met Asp Asn Ala Arg Val Val Asp Gln Leu
                165                 170                 175

Met Lys Asp Leu Gly Phe Gly Ser Gly Tyr Ile Ile Gln Gly Gly Asp
                180                 185                 190

Ile Gly Ser Phe Val Gly Arg Leu Leu Gly Val Gly Phe Asp Ala Cys
        195                 200                 205

Lys Ala Val His Leu Asn Leu Cys Ala Met Arg Ala Pro Pro Glu Gly
        210                 215                 220

Pro Ser Ile Glu Ser Leu Ser Ala Ala Glu Lys Glu Gly Ile Ala Arg
225                 230                 235                 240

Met Glu Lys Phe Met Thr Asp Gly Leu Ala Tyr Ala Met Glu His Ser
                245                 250                 255

Thr Arg Pro Ser Thr Ile Gly His Val Leu Ser Ser Ser Pro Ile Ala
                260                 265                 270

Leu Leu Ala Trp Ile Gly Glu Lys Tyr Leu Gln Trp Val Asp Lys Pro
        275                 280                 285

Leu Pro Ser Glu Thr Ile Leu Glu Met Val Ser Leu Tyr Trp Leu Thr
        290                 295                 300

Glu Ser Phe Pro Arg Ala Ile His Thr Tyr Arg Glu Thr Thr Pro Thr
305                 310                 315                 320

Ala Ser Ala Pro Asn Gly Ala Thr Met Leu Gln Lys Glu Leu Tyr Ile
                325                 330                 335

His Lys Pro Phe Gly Phe Ser Phe Phe Pro Lys Asp Leu Cys Pro Val
                340                 345                 350
```

```
Pro Arg Ser Trp Ile Ala Thr Thr Gly Asn Leu Val Phe Phe Arg Asp
        355                 360                 365

His Ala Glu Gly Gly His Phe Ala Ala Leu Glu Arg Pro Arg Glu Leu
        370                 375                 380

Lys Thr Asp Leu Thr Ala Phe Val Glu Gln Val Trp Gln Lys
        385                 390                 395
```

<210> 6
<211> 157
<212> PRT
<213> Homo sapiens

<400> 6

```
Val Arg Ser Ser Ser Arg Thr Pro Ser Asp Lys Pro Val Ala His Val
1               5                   10                  15

Val Ala Asn Pro Gln Ala Glu Gly Gln Leu Gln Trp Leu Asn Arg Arg
            20                  25                  30

Ala Asn Ala Leu Leu Ala Asn Gly Val Glu Leu Arg Asp Asn Gln Leu
            35                  40                  45

Val Val Pro Ser Glu Gly Leu Tyr Leu Ile Tyr Ser Gln Val Leu Phe
        50                  55                  60

Lys Gly Gln Gly Cys Pro Ser Thr His Val Leu Leu Thr His Thr Ile
65                  70                  75                  80

Ser Arg Ile Ala Val Ser Tyr Gln Thr Lys Val Asn Leu Leu Ser Ala
                85                  90                  95

Ile Lys Ser Pro Cys Gln Arg Glu Thr Pro Glu Gly Ala Glu Ala Lys
            100                 105                 110

Pro Trp Tyr Glu Pro Ile Tyr Leu Gly Gly Val Phe Gln Leu Glu Lys
            115                 120                 125

Gly Asp Arg Leu Ser Ala Glu Ile Asn Arg Pro Asp Tyr Leu Asp Phe
        130                 135                 140

Ala Glu Ser Gly Gln Val Tyr Phe Gly Ile Ile Ala Leu
145                 150                 155
```